(19) Europäisches Patentamt / European Patent Office / Office européen des brevets

(11) **EP 3 877 049 B1**

(12) **EUROPÄISCHE PATENTSCHRIFT**

(45) Veröffentlichungstag und Bekanntmachung des Hinweises auf die Patenterteilung:
**16.04.2025 Patentblatt 2025/16**

(21) Anmeldenummer: **19831975.8**

(22) Anmeldetag: **06.11.2019**

(51) Internationale Patentklassifikation (IPC):
*A61N 5/10* (2006.01)

(52) Gemeinsame Patentklassifikation (CPC):
**A61N 5/1049; A61N 5/1045; A61N 5/1047; A61N 5/1067; A61N 5/107**

(86) Internationale Anmeldenummer:
**PCT/DE2019/000291**

(87) Internationale Veröffentlichungsnummer:
**WO 2020/094167 (14.05.2020 Gazette 2020/20)**

(54) **VERFAHREN ZUR ECHTZEITBEZOGENEN KORREKTUR DER RÄUMLICHEN LAGE DES ZENTRALSTRAHLES VON STRAHLENTHERAPIEGERÄTEN UND DER PATIENTENPOSITION**

METHOD FOR REAL-TIME-RELATED CORRECTION OF THE SPATIAL POSITION OF THE CENTRAL BEAM OF RADIOTHERAPY DEVICES AND OF THE PATIENT POSITION

PROCÉDÉ DE CORRECTION EN TEMPS RÉEL DE LA POSITION SPATIALE DU FAISCEAU CENTRAL D'APPAREILS DE RADIOTHÉRAPIE ET DE LA POSITION DU PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.11.2018 DE 102018008806**

(43) Veröffentlichungstag der Anmeldung:
**15.09.2021 Patentblatt 2021/37**

(73) Patentinhaber: **Städtisches Klinikum Dessau**
**06847 Dessau-Roßlau (DE)**

(72) Erfinder: **WÖSLE, Markus**
**06844 Dessau-Roßlau (DE)**

(74) Vertreter: **Däbritz, Frank**
**Dinter Kreißig & Partner**
**Gottschedstraße 12**
**04109 Leipzig (DE)**

(56) Entgegenhaltungen:
**WO-A2-89/05171 US-A1- 2005 228 255**
**US-A1- 2014 288 349**

**Beschreibung**

Die Erfindung betrifft ein Verfahren zur echtzeitbezogenen Korrektur der räumlichen Lage des Zentralstrahles von Strahlentherapiegeräten und Therapiesimulatoren sowie der Patientenposition. Insbesondere bezieht sich das Verfahren auf die echtzeitbezogene Korrektur von Bestrahlungspositionen des Collimators und/oder der Position des Patientenlagerungstisches während der Durchführung der Strahlentherapie beziehungsweise der Therapiesimulation. Anstatt der Bestrahlungspositionen des Collimators kann auch die Bestrahlungsposition der Strahlungsquelle korrigiert werden. Das Verfahren kommt bevorzugt bei der Nutzung von präzisen Bestrahlungsmethoden wie der stereotaktischen Radiotherapie und Radiochirurgie zur Anwendung; es ist jedoch bei jeder Bestrahlungstechnik und bei der Therapiesimulation anwendbar.

Aus dem bekannten Stand der Technik können Lösungen entnommen werden, die sich auf die Ermittlung der Position von Objekten in einem Bestrahlungsraum beziehen. So wird nach der EP 2 883 568 A1 ein System und ein Verfahren zur Ermittlung der Position von Objekten in einem Bestrahlungsraum vorgestellt. Dazu werden im Bestrahlungsraum Raumlaser angeordnet, die auf die Oberfläche eines auf einem Patiententisch befindlichen Patienten mindestens eine Laserlinie projizieren. Mittels mindestens einer Kamera werden dann die auf der Oberfläche des Patienten projizierten Laserlinien erfasst. Den Kameras ist eine Auswerte- und Steuereinrichtung nachgeordnet, die während eines Bestrahlungsvorganges auf Grundlage der von der Kamera erfassten Messwerte durch ein Echtzeit-Triangulationsverfahren die Koordinatenpunkte der projizierten Laserlinie ermittelt. Die ermittelten Koordinatenpunkte werden mit Sollkoordinatenpunkten verglichen und die erhaltenen Werte gespeichert. Die gespeicherten Werte dienen der Dokumentation des Bestrahlungsvorgangs.

Eine weitere Lösung nach der DE 101 47 633 A1 betrifft ein System und ein Überwachungsverfahren, mit denen das Bestrahlungsziel, dessen Position und die Bewegung des Bestrahlungszieles erkannt werden. Mit diesem vorgeschlagenen System soll zum einen die durch den dabei erforderlichen Kopfring und das Mundstück auf den Patienten ausgeübte Belastung herabgesetzt werden. Zum anderen soll eine genaue Strahlentherapie nicht nur im Kopfbereich, sondern auch im Rumpfbereich ermöglicht werden. Zudem soll bei der Therapie kein Positionierungsrahmen für das Bestrahlungsziel erforderlich sein. Dazu kommt u.a. eine Echtzeit-Abbildungsvorrichtung zur Aufnahme von hochaufgelösten 3D-Bildern des Bestrahlungszielbereichs zur Anwendung. Mittels einer Bestrahlungsbedingungs-Korrekturvorrichtung werden die Position und die Richtung der Bestrahlungszielbereiche in Echtzeit anhand der gewonnenen Bilder geändert.

Bei einer weiteren Lösung nach der DE 10 2010 041 752 B4 werden ein Verfahren zur Kalibrierung eines Lamellenkollimators sowie ein dazu erforderliches Computerprogramm beschrieben. Derartige Kollimatoren umfassen eine Vielzahl von Lamellen, die unabhängig voneinander bewegbar sind. Durch den auf diese Weise flexibel einstellbaren Strahlungsbereich wird der Strahl auf das betroffene, zu bestrahlende Gewebe beschränkt. Mit dem vorgestellten Verfahren wird die geometrische Kalibrierung des Gesamtsystems aus Lamellen und Lamellenträger aufwandsärmer gestaltet. Erreicht wird dies durch Ermittlung der Positionsabweichungen für die Lamellen durch eine direkte Anwendung von Bildgebungsverfahren. Die Positionsabweichungen des Lamellenträgers können dabei ohne explizite Messungen bestimmt werden. Durch die Aufnahme der Lamellen und einen Vergleich der Aufnahme mit einem Referenzbild werden Abweichungen der Lamellenpositionen bestimmt.

Bekannt sind weiterhin nach der WO 2013/041720 A1 ein System und ein Verfahren zur Positionierung von Patienten mittels nuklearer Bildgebung. Dazu wird die Position von radioaktiv markiertem Zielgewebe von Patienten bestimmt. In Abhängigkeit von Messdaten eines Nuklearstrahlungs-Detektors wird danach durch eine Vorrichtung die relative Position des Patienten zu einer Strahlungsquelle eingestellt. Dabei ist eine präzise Erfassung der Zielstruktur für die möglichst geringe Schädigung des gesunden umliegenden Gewebes erforderlich. Mittels des Detektorsystems und einer nachgeschalteten rechnerischen Verarbeitung der erfassten Detektorsignale werden Informationen über die Größe, Position und die Form des Zielgewebes im Raum ermittelt. Durch Vergleich dieser Daten mit den geometrischen Daten des Strahlengangs des Bestrahlungsgeräts lässt sich ermitteln, wie die Position des Patienten verändert werden muss. Im einfachsten Fall stellt die Korrekturgröße einen dreidimensionalen Vektor dar, dessen Länge und Richtung die benötigte Veränderung angeben, damit eine Bestrahlung begonnen werden kann.

Mit der EP 1 740 098 B1 wird eine Patientenpositionierungsanordnung beschrieben, die als Robotervorrichtung ausgebildet ist und zum Stützen und Verschieben des aufgenommenen Patienten dient. Die zur Anwendung kommende Robotervorrichtung ist in der Lage, den Patienten in mindestens fünf Freiheitsgraden zu verschieben. Dazu besitzt die Vorrichtung eine Armanordnung, die an den Behandlungstisch gekoppelt ist, um den Behandlungstisch um drei Drehfreiheitsgrade sowie in drei Translationsfreiheitsgraden zu versetzen. Über ein Sensorsystem wird die Position der Robotervorrichtung relativ zum Behandlungskoordinatensystem erfasst. Dazu werden Fast-Echtzeitdaten des Behandlungsziels und Informationen über die Position der therapeutischen Strahlenquelle genutzt und die Verschiebung der Robotervorrichtung gesteuert. Mittels der Steuerung wird das Behandlungsziel nach der Position der therapeutischen Strahlenquelle ausgerichtet.

Der nach der DE 20 2012 013 430 U1 bekannt gewordene Gegenstand betrifft eine therapeutische Vorrichtung zur Behandlung eines vorgegebenen Körperteiles eines Patienten mit Strahlen. Das dabei genutzte Verfahren dient ebenfalls zur Steuerung der therapeutischen Vorrichtung unter Verwendung eines Echtzeitsystems. Die Baugruppen des mit dem Positionierungssystem des Behandlungstisches, des Strahlenpositionierungssystems und des Kollimators verbundenen Echtzeitsystems sind programmierbar ausgebildet.

Weiterhin aus dem Stand der Technik bekannt geworden ist nach der DE 10 2011 082 257 B4 ein Strahlentherapiegerät mit einer Bestrahlungsfeldbegrenzungsvorrichtung, die einen Multi-Leaf-Collimator (MLC) zur Einstellung des Bestrahlungsfeldes, einen Patiententisch und eine Verifikationsvorrichtung zum visuellen Verifizieren des Bestrahlungsfeldes aufweist. Die Verifikationsvorrichtung ist dabei so ausgebildet, dass das Bestrahlungsfeld auf einem Patienten optisch angezeigt wird. Der vom Isozentrum des Strahlentherapiegerätes beabstandet positionierte Patient befindet sich dazu in einem virtuellen Isozentrum. Mittels des Patiententisches wird der Patient vom virtuellen Isozentrum im Isozentrum des Strahlentherapiegerätes in Position gebracht. Das virtuelle Isozentrum des Strahlentherapiegerätes und eine Projektion des Bestrahlungsfeldes werden dabei miteinander kombiniert und auf diese Weise eine Optimierung im Arbeitsablauf innerhalb der Radioonkologie erzielt.

Alle vorbeschriebenen Lösungen sind für die Herstellung und Anwendung komplett neu gestalteter Strahlentherapiegeräte mit den damit verbundenen hohen Anschaffungskosten vorgesehen. Keines der Verfahren in den oben beschriebenen Patenten korrigiert im Gegensatz zum eingangs erwähnten Verfahren Zentralstrahlabweichungen des Strahlentherapiegeräts, die durch mechanische Toleranzen und elastische Verformungen durch Eigengewicht verursacht werden. Die oben zitierten Patente sehen das Isozentrum eines Strahlentherapiegeräts als punktförmig an, obwohl es immer eine räumliche Ausdehnung besitzt.

Bei vielen im klinischen Einsatz befindlichen medizinischen Elektronen-Linearbeschleunigern führen die Durchbiegung des Tragarms infolge Eigengewichts sowie isozentrische Rotationen des Patientenlagerungstischs infolge mechanischer Toleranzen zu räumlichen Zentralstrahlverlagerungen relativ zum Tumorzentrum. Die betragsmäßig größte Komponente der Zentralstrahlverlagerung in Y-Richtung wird regelmäßig durch die elastische Verformung des Tragarms, der den Strahlerkopf aufnimmt, verursacht; dieses Phänomen wird in der Fachliteratur "Gantry Sag" genannt. Durch die Steifigkeitserhöhung der Tragarmkonstruktionen versuchte man bisher, Zentralstrahlverlagerungen zu minimieren. So sind verbesserte Konstruktionen des Tragarms und der Tragarmlagerung bezüglich Steifigkeit bei allen Herstellern von Strahlentherapiegeräten gegen einen Aufpreis erhältlich. Laut den Spezifikationen der Linearbeschleuniger-Hersteller besitzt das globale Isozentroid (das Isozentrum mit räumlicher Ausdehnung) mit diesen verbesserten Tragarmkonstruktionen einen maximalen Durchmesser von 1.5 mm. Bei Standardausführungen beträgt der maximale Durchmesser 2 mm.

Bei den präzisen Bestrahlungstechniken wie stereotaktische Radiotherapie beziehungsweise stereotaktische Radiochirurgie von Krankheitsherden in unmittelbarer Nachbarschaft zu

- strahlensensiblen seriellen Organen, die lebenswichtig sind, wie beispielsweise am Hirnstamm und am Rückenmark beziehungsweise

- Organen, die mit ihrem Funktionserhalt eine organerhaltende Therapie erst ermöglichen, wie beispielsweise einer Tränendrüse bei der organerhaltenden Augentumortherapie,

wünscht sich der behandelnde Arzt regelmäßig ein Strahlentherapiegerät mit einem idealen Isozentrum. Damit wäre er in der Lage, die lokalen Tumorkontrollwahrscheinlichkeiten bei verminderten Organtoxizitäten zu erhöhen, indem er weniger gesundes Gewebe bestrahlen muss.

Die Aufgabe der Erfindung besteht deshalb in der Schaffung eines Verfahrens, mit dem die Positionsabweichungen des Zentralstrahles vorhandener Strahlentherapiegeräte verringert und dabei der für die Genauigkeit und den Patientenkomfort erforderliche technische Aufwand optimiert wird.

Gelöst wird diese Aufgabe mit dem geschaffenen Verfahren gemäß den beschreibenden Merkmalen nach Patentanspruch 1. Vorteilhafte Weiterbildungen des Verfahrens werden durch die Merkmale der Patentansprüche 2 bis 16 beschrieben.

Vorteilhaft wird die Erhöhung der echtzeitbezogenen Genauigkeit des Zentralstrahles durch die Steuerung der räumlichen Positionen der Collimatorelemente (Blockblenden, Lamellen, Lamellenträger, Rundcollimatoren) beziehungsweise der Komponenten der Strahlungsquelle und/oder des Patientenlagerungstischs für optional weitere Freiheitsgrade der Korrekturbewegungen erreicht. Der optimale technische Aufwand wird durch funktionelle Zuschaltungen von separaten Steuerbaugruppen zu den Steuereinrichtungen vorhandener Strahlentherapiegeräte erzielt. Günstig für eine Senkung des materiell technischen Aufwandes für die Änderungen der räumlichen Positionen der Collimatorelemente beziehungsweise der Strahlungsquelle und/oder des Patientenlagerungstischs innerhalb der optional weiteren Freiheitsgrade erweisen sich die Anwendungen separater Verstelleinrichtungen. Dazu sind die separaten Verstelleinrichtungen über die jeweiligen Steuerbaugruppen mit dem Collimator beziehungsweise der Strahlungsquelle und/oder dem Patientenlagerungstisch verbunden. Effektvoll wird der technische und kostenmäßige Aufwand für die Steuerung der räumlichen Position gesenkt, wenn die Steuerung von einer Steuerbaugruppe ausgeführt wird, die als ein Microcontroller ausgebildet ist, der ein die Verstelleinrichtungen steuerndes Computerprogramm enthält. Die Optimierung der Steuerung bereits vorhandener Strahlentherapiegeräte wird wirkungsvoll dadurch erreicht, wenn mittels der zuschaltbaren Steuerbaugruppen die Steuerung der Collimatorelemente in *X*- und *Y*-Richtung des collimatorfesten Koordinatensystems beziehungsweise der Strahlungsquellenkomponenten in *X*- und *Y*-Richtung des quellenfesten Koordinatensystems und/oder des Patientenlagerungstisches innerhalb der Freiheitsgrade in alle drei Raumrichtungen des tischfesten Koordinatensystems erfolgt. Zwecks Erzielung einer hohen Positionierungsgenauigkeit und eines maximalen Patientenkomforts wird vorzugsweise die Steuerung innerhalb der Freiheitsgrade in *X*- und *Y*-Richtung auf die echtzeitbezogenen Positionsänderungen der Collimatorelemente beschränkt. Minimale Kosten durch die Vermeidung der Neukonstruktion zusätzlicher Freiheitsgrade werden wirkungsvoll dann erreicht, wenn die Steuerung innerhalb der Freiheitsgrade in *X*-, *Y*- und Z-Richtung auf die echtzeitbezogene Positionsänderung des Patientenlagerungstisches beschränkt ist. Abhängig vom festgelegten Optimierungsziel

- maximaler Patientenkomfort bei unbewegter Tischplatte,

- minimale Kosten der Neukonstruktion oder Umrüstung,

- minimale effektive Restabweichung des Zentralstrahls relativ zum Zentrum des Krankheitsherds,

- maximale geometrische Stabilität des Zentralstrahls über der Zeit oder

- minimaler Zeitaufwand für Verifikationen und Neukalibrierungen

kann die Steuerung des Strahlentherapiegeräts jede beliebige Kombination der echtzeitbezogenen Positionsänderungen der Collimatorelemente, der Strahlungsquellenkomponenten und des Patientenlagerungstischs ausführen.

Nachfolgend soll die Erfindung anhand eines Ausführungsbeispiels näher erläutert werden. Die Zeichnungen zeigen:

**Figur 1:** Neukonstruktion des Freiheitsgrades in *Y*-Richtung für den Multi-Leaf-Collimator.

**Figur 2:** Neukonstruktion der Freiheitsgrade in *X*- und *Y*-Richtung für die Rundcollimatoren.

**Figur 3:** Darstellungen der Abweichungen der Zentralstrahlposition.

**Figur 4:** Translatorische Geschwindigkeitskomponenten des Patientenlagerungstischs.

**Figur 5:** Translatorische Beschleunigungskomponenten und resultierende Beschleunigung des Patientenlagerungstischs.

**Figur 6:** Translatorische Ruckkomponenten des Patientenlagerungstischs bei der Korrektur der Patientenposition.

Eine typische Tragarmkonstruktion aus Stahl eines Linearbeschleunigers besteht aus mindestens zwei Schweißteilen, die miteinander zu einem L-förmigen Tragarm verschraubt werden. Die bevorzugte Profilform ist U-förmig mit hohem Steg; die Profile werden paarweise angeordnet. Der Raum zwischen den Profilen besitzt dadurch einen rechteckigen Querschnitt, in dem zahlreiche elektrische, elektronische und physikalische Baugruppen der Strahlerzeugung, Energieauswahl, Strahlformung und Dosisleistungsregelung untergebracht sind. Der drehbare Tragarm selbst wird über ein vorgespanntes Vierpunkt-Rillenkugellager im Stativ des Strahlentherapiegeräts gelagert. Nachfolgend beschriebene Verbesserungen der vorherrschenden Tragarmkonstruktion basieren auf der Analyse der elastischen Durchbiegung eines einseitig fest eingespannten Trägers, der durch eine Streckenlast, die seinem Eigengewicht entspricht, statisch belastet wird. Dies ist ein einfaches Modell für einen Tragarm. Im Standardwerk [1] findet man dazu den Neigungswinkel

$$\alpha_x = \frac{q_z \cdot L^3}{6 \cdot E \cdot I_z} = \frac{m' \cdot g \cdot L^3}{6 \cdot E \cdot I_z} = \frac{\rho \cdot V \cdot g \cdot L^2}{6 \cdot E \cdot I_z} \tag{1}$$

um die Biegeachse in der Querrichtung x am freien Ende, der direkt proportional zu einer damit zusammenhängenden Zentralstrahlverlagerung senkrecht zur Biegeachse ist. Damit ist gemäß Gleichung (1) die Zentralstrahlverlagerung

proportional zur Streckenlast $q_x = m' \cdot g$ in z-Richtung beziehungsweise zur Masse $m'$ pro Länge, die proportional zur physikalischen Dichte $p$ ist, und zur 3. Potenz der Trägerlänge L sowie umgekehrt proportional zum Elastizitätsmodul E des Werkstoffs und zum axialen Flächenmoment 2. Grades $I_x$ des Profils um die x-Achse. Die Konstante g ist die Fallbeschleunigung im Gravitationsfeld der Erde; $V$ ist das Volumen des Trägers.

Nützlich sind zur weiteren Verringerung des Neigungswinkels $\alpha_x$ folgende Maßnahmen:

• Weglassen aller nicht notwendigen, tragenden Schraubverbindungen, um deren Setzbeträge, die zur elastischen Verformung des Tragarms beitragen, zu eliminieren. Form- und kraftschlüssige Schraubverbindungen, die eine hohe Steifigkeit besitzen, haben außerdem vergleichsweise mehr Masse als stoffschlüssige Schweißverbindungen.

• Erhöhung der Biegesteifigkeit des Tragarms durch verbesserte Formgebung der tragenden Profile, ohne dabei die Bauhöhe und die Masse pro Länge zu erhöhen. Das heißt, es wird ein Profil gesucht, bei dem der Quotient $m' / I_x$ minimal wird. Hier sind schmale I-Träger gemäß DIN 1025 - Blatt 1 und mittelbreite I-Träger der Form *IPE* gemäß DIN 1025 - Blatt 5 besser als U-Stahl gemäß DIN 1026 - Blatt 1 beziehungsweise UPE-Stahl gemäß DIN 1026 - Blatt 2. Bei einer Profilhöhe im Bereich (260 ... 270) mm, die in Tragarmkonstruktionen üblich ist, würde man die Biegesteifigkeit mit I-Trägern gegenüber einem U-Profil um bis zu 7.7 % bei nahezu unverändertem Metergewicht erhöhen können. Die für diese Abschätzung benutzten Kennwerte der Stahlträgerprofile entnehme man Tabelle 1.

| Profil | $H$ [mm] | $m'\left[\dfrac{kg}{m}\right]$ | $I_x$ [cm$^4$] | $\dfrac{m'}{I_x}\left[\dfrac{g}{m \cdot cm^4}\right]$ | $\left(\dfrac{m'}{I_x}\right)_{rel}$ [1] | DIN |
|---|---|---|---|---|---|---|
| U | 260 | 37.9 | 4820 | 7.863 | 1.077 | 1026 – 1 |
| I | 260 | 41.9 | 5740 | 7.300 | 1.000 | 1025 – 1 |
| UPE | 270 | 35.2 | 5255 | 6.698 | 1.074 | 1026 – 2 |
| IPE | 270 | 36.1 | 5790 | 6.235 | 1.000 | 1025 – 5 |

• Neben der Geometrie können auch die Werkstoffeigenschaften optimiert werden, damit der Quotient $m' / E$ beziehungsweise $\rho / E$ minimal wird. Anstatt Stahl wäre hier Molybdän besser; sein Verhältnis $\rho / E$ ist um 18.3 % geringer wie Tabelle 2 zeigt. Diese Überlegung ist nur theoretischer Art, da Molybdän viel teurer und schwieriger zu verarbeiten als Stahl ist. Der Preis pro Kilogramm Halbzeug aus Molybdän ist um den Faktor 100 ... 240 höher. Es sind keine hochfesten Schweißungen herstellbar; Hartlöten ist jedoch möglich. Andere Werkstoffe mit hohem Elastizitätsmodul wie Nickel und Wolfram sind bei dieser Abschätzung schon aus rein mechanischer Sicht nicht zielführend (siehe Werte für $\rho / E$ in Tabelle 2).

| Werkstoff | $\rho\left[\dfrac{kg}{dm^3}\right]$ | $E\left[\dfrac{N}{mm^2}\right]$ | $\dfrac{\rho}{E}\left[\dfrac{g \cdot mm^2}{dm^3 \cdot N}\right]$ | $\left(\dfrac{\rho}{E}\right)_{rel}$ [1] |
|---|---|---|---|---|
| Stahl | 7.95 | 210000 | 0.038 | 1.000 |
| Nickel | 8.8 | 210000 | 0.042 | 1.107 |
| Molybdän | 10.21 | 330000 | 0.031 | 0.817 |
| Wolfram | 19.3 | 390000 | 0.049 | 1.307 |

• Da auch die Steifigkeit des Tragarmlagers die Zentralstrahlverlagerung beeinflusst, würde man das einreihige Rillenkugellager durch ein zweireihiges Kegelrollenlager in X- oder O-Anordnung ersetzen. Aus der Sicht des Konstrukteurs würde man hier die O-Anordnung bevorzugen, da sie ein geringeres Kippspiel aufweist und damit starrer als eine X-Anordnung ist. Die paarigen Lageranordnungen erhöhen die statische und dynamische Steifigkeit einer Wälzlagerung beträchtlich. Außerdem ist durch den Linienkontakt zwischen den kegelförmigen Wälzkörpern und den Laufringen die Hertzsche Pressung geringer und die Lagersteifigkeit höher als beim Punktkontakt kugelförmiger Wälzkörper. Die statischen Tragzahlen dieser Wälzlagertypen mit einem jeweiligen Bohrungsdurchmesser in der Größenordnung der tatsächlich verwendeten Tragarmlager in Tabelle 3 belegen dies. Bei unveränderten Lagerkräften und -drehmomenten wäre eine Erhöhung der Lagersteifigkeit um den Faktor 7.5 bis 8.7 möglich. Dies ist eine grobe Abschätzung mittels der Verhältnisse der statischen Tragzahlen. Die statische Tragzahl eines Wälzlagers entspricht derjenigen ruhenden Lagerkraft, mit der an den Kontaktstellen zwischen den Wälzkörpern und den Laufringen bleibende Verformungen auftreten, die kleiner oder gleich dem $10^{-4}$-fachen des Wälzkörperdurchmessers sind.

**Tabelle 3:** Bohrungsdurchmesser $d$, Außendurchmesser $D$, Lagerbreite $B$, statische Tragzahl $C_0$, statische Tragzahl $C_{0,rel}$ relativ zum Rillenkugellager und Masse $m$ unterschiedlicher Wälzlagerbauformen der Firma SKF GmbH, Schweinfurt, Deutschland (TDO = O-Anordnung, TDI = X-Anordnung; Lagerkurzzeichen: [1]609/710 MA, [2]BT2B 328028/HA1, [3]331198).

| Wälzlagertyp | $d$ [$mm$] | $D$ [$mm$] | $B$ [$mm$] | $C_0$ [$kN$] | $C_{0.rel}$ [1] | $m$ [$kg$] | Bauform | Anordnung |
|---|---|---|---|---|---|---|---|---|
| Rillenkugellager[1] | 710.0 | 950.0 | 78.0 | 1290 | 1.000 | 167 | einreihig | - |
| Kegelrollenlager[2] | 711.2 | 914.4 | 190.5 | 9650 | 7.481 | 265 | zweireihig | TDO |
| Kegelrollenlager[3] | 660.4 | 812.8 | 176.2 | 11200 | 8.682 | 195 | zweireihig | TDI |

Wollte man die Biegesteifigkeit einer Tragarmkonstruktion noch weiter verbessern, sollte man also die Formgebung der tragenden Stahlprofile sowie die Wälzlagerung des Tragarms ändern. Das Tragwerk des Tragarms sollte als ein Schweißteil ohne Schraubverbindungen gefertigt werden.

Der resultierende Steifigkeitsgewinn bei verbesserter Lager- und Tragarmsteifigkeit wird durch die Reihenanordnung beider Steifigkeiten stark geschmälert. Die Gesamtsteifigkeit $c_{ges}$ lässt sich gemäß der Gleichung

$$\frac{1}{c_{ges}} = \frac{1}{c_1} + \frac{1}{c_2} \tag{2}$$

aus den Einzelsteifigkeiten $c_1$ und $c_2$ ermitteln. Damit ergibt sich die relative Gesamtsteifigkeit vor der Verbesserung zu 0.5 mit den relativen Grundsteifigkeiten $c_1 = c_2 \equiv 1$, unter der Annahme, dass die absoluten Einzelsteifigkeiten dieselbe Größenordnung aufweisen. Mit den relativen Steifigkeitserhöhungen $c_1 = 7.5$ und $c_2 = 1.077$ durch das zweireihige Kegelrollenlager in O-Anordnung beziehungsweise durch den Einsatz schmaler I-Träger in der Tragarmkonstruktion ergäbe sich die relative Gesamtsteifigkeit zu 0.941. Man könnte mit den konstruktiven Verbesserungen die Durchbiegung des Tragarms unter Eigengewicht und somit die Zentralstrahlverlagerung allenfalls halbieren, so wie es der Quotient $c_{ges}|_{vorher}/ c_{grs}|_{nachher} = 0.5 / 0.941 = 0.531$ vorgibt.

Einen Lösungsversuch zur geometrischen Zentralstrahlkorrektur beschreiben die Autoren der Publikation [2], indem sie das Isozentroid des Tragarms für eine Vollrotation messtechnisch bestimmen. Sie benutzen lediglich die tragarmwinkelabhängigen Zentralstrahlabweichungen in $Y$-Richtung, um das Isozentroid auch nur in dieser Richtung verkleinern zu können. Anschließend werden die Lamellenpositionen des Multi-Leaf-Collimators tragarmwinkelabhängig, offline (im Bestrahlungsplanungssystem) um die Zentralstrahlabweichungen korrigiert. Der Collimatorwinkel muss dabei auf konstant 90° eingestellt werden. Auf diese Weise können Tumoren in Patienten nicht optimal bestrahlt werden, da der optimale Collimatorwinkel von der Einstrahlrichtung abhängt.

Bei den Hardware-Lösungen zur Verringerung der mechanischen Zentralstrahlverlagerungen werden die Tragarme und Tragarmlager von Elektronen-Linearbeschleunigern steifer gebaut. Man ist jedoch im Bauraum eingeschränkt. Außerdem ist eine Steifigkeitserhöhung mit dem klassischen Maschinenbau-Werkstoff Stahl nur begrenzt durch Formgebung möglich, sodass man mit einer Erhöhung des Eigengewichts konfrontiert wird; hier ist man mittlerweile bei über 7 t angekommen. Die Tragarm- und Tragarmlagersteifigkeit, die zusammen mit der großen bewegten Masse hauptsächlich die Zentralstrahlpositionsabweichungen verursachen, bleiben aber immer endlich.

Mit der Erfindung kann an dem Stand der Technik angeknüpft werden, bei dem durch Versteifungen des Tragarms und/oder des Tragarmlagers im Stativ die Zentralstrahlpositionsabweichungen mehr oder weniger reduziert werden. Mittels Messungen, Berechnungen sowie Steuerungs- und Regelungsalgorithmen sollen die verbleibenden Zentralstrahlpositionsabweichungen weiter verringert beziehungsweise kompensiert werden, falls dies die Toleranzbreiten und Reproduziergenauigkeiten der Stellglieder des Strahlentherapiegeräts zulassen.

Da Präzision im Maschinenbau teuer ist, ist man mit der Erfindung in der Lage, aus jedem Standard-Strahlentherapiegerät, das einen weniger steifen Tragarm als ein Stereotaxie-Strahlentherapiegerät besitzt, ein Stereotaxie-Strahlentherapiegerät zu machen. Die weitere Entwicklung steiferer Tragarmkonstruktionen ist damit obsolet. Mithilfe der Erfindung können geometrisch präzise Strahlentherapiegeräte billiger hergestellt werden, da konstruktive Maßnahmen zur Erhöhung der Tragarmsteifigkeit in die umgekehrte Richtung gehen können: Man kann die gesamte Tragarmkonstruktion in Zukunft leichter bauen und größere geometrische Abweichungen des Zentralstrahls als bisher zulassen. Außer der Kosteneinsparung wären weniger Probleme bei der Einbringung und bei der Montage der schweren Maschinenteile vor Ort zu erwarten, da oft zulässige Deckenbelastungen und lichte Weiten der Zugangswege in bestehenden Gebäuden begrenzt sind.

Es gibt zwei Wege zu einer korrigierten Bestrahlungsgeometrie:

1. Man bringt den realen Zentralstrahl, der von seiner idealen Position abweicht, ins ideale Isozentrum.
2. Man bringt das Zentrum des Krankheitsherds mit dem Patienten zum Durchstoßpunkt des realen Zentralstrahls in der Isozentrumsebene.

Beide Methoden können auch kombiniert werden (abhängig vom festgesetzten Optimierungsziel, siehe oben). Als Resultat erhält man immer die bestmögliche Bestrahlungsgeometrie, die im Rahmen der verbleibenden Toleranzbreiten und endlichen Reproduziergenauigkeiten der Stellglieder des Strahlentherapiegeräts möglich ist.

**Durchführung der Zentralstrahlpositionskorrektur**

Die räumliche Zentralstrahlabweichung relativ zum Messkörper, der beim Winston-Lutz-Test das ideale Isozentrum anzeigt, im Inertialsystem $I = \{ISO, X, Y, Z\}$ ist im Vektor

$$_I \mathbf{r}_{ISO}(K) = \begin{pmatrix} _I X_{ISO}(K) \\ _I Y_{ISO}(K) \\ _I Z_{ISO}(K) \end{pmatrix} \in \mathbb{R}^3 \tag{3}$$

enthalten. Gleichung (3) gilt für die drei Winkel $K \in \{G, C, T\}$ des Tragarms, Collimators und Patientenlagerungstischs. Die Koordinatenachsen sind wie folgt definiert:

- $X$ zeigt von links nach rechts vom Fußende der Patientenlagerungstischplatte aus betrachtet,
- $Y$ zeigt in der Längsrichtung der Patientenlagerungstischplatte in die Richtung der Tragarmlagerung und
- $Z$ zeigt vom Boden in Richtung der Decke des Bestrahlungsraums.

Der Vektor in Gleichung (3) beschreibt das Isozentroid vollständig. Es wird zunächst in der Ebene des Electronic Portal Imaging Device (EPID), das zur Messung verwendet wird, im dortigen Koordinatensystem *EPID* gemäß

$$_{EPID}\mathbf{r}_{ISO} = {}_{EPID}\mathbf{r}_{CAX} - {}_{EPID}\mathbf{r}_{MK} \tag{4}$$

aus den Ortsvektoren r des Zentralstrahls *CAX* und des Messkörpers *MK* berechnet. Die im Folgenden benötigten Abbildungsmatrizen

$$\mathbf{A}_\beta = \begin{pmatrix} \cos\beta & 0 & \sin\beta \\ 0 & 1 & 0 \\ -\sin\beta & 0 & \cos\beta \end{pmatrix} \in \mathbb{R}^{3,3} \text{ und} \tag{5}$$

$$\mathbf{A}_\gamma = \begin{pmatrix} \cos\gamma & -\sin\gamma & 0 \\ \sin\gamma & \cos\gamma & 0 \\ 0 & 0 & 1 \end{pmatrix} \in \mathbb{R}^{3,3} \tag{6}$$

sowie deren inverse Matrizen, um von um die *Y*-Achse beziehungsweise Z-Achse gedrehten körperfesten Koordinatensystemen ins raumfeste Inertialsystem zu gelangen und umgekehrt, entnehme man zum Beispiel dem Buch [3]. $\beta$ und $\gamma$ sind dabei die vorzeichenbehafteten Drehwinkel um die *Y*- beziehungsweise Z-Achse. Die Messung erfolgt mit einem genügend kleinen Winkelinkrement für alle drei Winkelfreiheitsgrade G, C und T. Zur Erzeugung eines geschlossenen Kennfelds zur Beschreibung der globalen Zentralstrahlabweichungen wird zwischen Winkelschritten nichtlinear interpoliert, beispielsweise mittels kubischer Splines beziehungsweise stückweise mittels kubischer Hermite-Interpolation. Idealerweise werden die Winkelbereiche von *G*, *C* und *T* stufenlos mit konstanter Winkelgeschwindigkeit durchfahren. In diesem Fall entfallen die Interpolationen.

Da die Isozentroide eines Strahlentherapiegeräts des Weiteren von den Parametern

- Strahlungsart,

- Teilchenenergie und

- Collimierungsart

abhängen, muss für jede zur Patientenbestrahlung angewendete Kombination dieser Parameter ein solches Kennfeld messtechnisch ermittelt werden. Für einen typischen Elektronen-Linearbeschleuniger im Photonenbetrieb, der vier Teilchenenergien erzeugen kann und drei Collimierungsarten zulässt, wären zwölf Kennfelder aufzunehmen.

Damit die Messergebnisse repräsentativ für einen elastischen Tragarm und somit reproduzierbar sind, muss man der Relaxation des Tragarms infolge elastischer Verformungen, von Setzbeträgen sowie der Tribologie im fettgeschmierten Tragarmwälzlager wirkungsvoll begegnen. Dazu ist ein Bewegungsprogramm für den Tragarm erforderlich, das er vor dem Beginn einer Präzisionsmessung der Isozentroidgeometrie durchlaufen muss:

- Vorbereitung der Messung beim Tragarmwinkel $G$ = 0°. Bei dieser Winkelstellung wird der Messkörper am Patientenlagerungstisch montiert und mittels der raumfesten Laser beziehungsweise mittels eines vom Strahlentherapiegerät unabhängigen radiologischen Patientenpositionierungssystems justiert.

- Der Tragarm muss nun 3 ½ Vollrotationen absolvieren, bevor die Messung des Tragarm-Isozentroids beginnt.

- Bevor die Messung des Collimator-Isozentroids stattfindet, muss der Tragarm 5 *min* bei der Winkelstellung 0° ruhen.

- Anschließend muss der Tragarm ebenfalls jeweils 5 *min* bei den Winkelstellungen -30° und 30° ruhen, bevor der linksseitige und rechtsseitige Teil des Patientenlagerungstisch-Isozentroids bestimmt werden kann.

Damit können geräteunabhängig reproduzierbare Isozentroide erzielt werden, die sich bezüglich ihrer Größe und Lage höchstens um 0.02 *mm* unterscheiden, selbst dann, wenn der Tragarm nachts oder über ein Wochenende in einer bestimmten Ruhestellung verharrt war.

Nun die Schritte der Zentralstrahlkorrektur im Einzelnen:

1. Bestimmung der Größe und Lage aller Isozentroide als Funktion des Tragarm-, Collimatorbeziehungsweise Tischwinkels. Die Blockblenden, Lamellen beziehungsweise Rundcollimatoren befinden sich jeweils in der Sollposition, das heißt sie befinden sich in ihren nominellen Positionen der Bestrahlungsplanung, auf die noch keine Positionskorrekturen angewendet worden sind. Das Tragarm-Isozentroid ergibt sich zu

$$_I\mathbf{r}_{ISO}(G) = \mathbf{A}_\beta \cdot k^{-1} \cdot {}_{EPID}\mathbf{r}_{ISO}(G). \tag{7}$$

Das Collimator-Isozentroid ergibt sich zu

$$_I\mathbf{r}_{ISO}(C) = \mathbf{A}_\gamma \cdot k^{-1} \cdot {}_{EPID}\mathbf{r}_{ISO}(C). \tag{8}$$

Das Patientenlagerungstisch-Isozentroid ergibt sich zu

$$_I\mathbf{r}_{ISO}(T) = \mathbf{A}_\beta \cdot k^{-1} \cdot {}_{EPID}\mathbf{r}_{ISO}(G. T). \tag{9}$$

Dabei gilt $\beta$ = $G$, $\gamma$ = C und

$$k = \frac{SID}{SAD} > 1, \tag{10}$$

wobei *SID* der eingestellte Fokus-EPID-Abstand und *SAD* der feste Fokus-ISO-Abstand ist.

2. Zusammensetzen des globalen Kennfelds für alle winkelabhängigen Zentralstrahlabweichungen relativ zum Messkörper:

$$_I\mathbf{r}_{ISO}(G. C. T) = {}_I\mathbf{r}_{ISO}(G) + [{}_I\mathbf{r}_{ISO}(C) - {}_I\mathbf{r}_{ISO}(C_0)] \\ + [{}_I\mathbf{r}_{ISO}(T) - {}_I\mathbf{r}_{ISO}(T_0)]. \tag{11}$$

Dabei sind $C_0 = 0°$ und $T_0 = 0°$ die Collimator- und Tischwinkelstellung zur Messung des Tragarm-Isozentroids.

3. Abbildung des globalen Kennfelds in das collimatorfeste Koordinatensystem C:

$$_C\mathbf{r}_{ISO}(G, C, T) = \mathbf{A}_\gamma^{-1} \cdot \mathbf{A}_\beta^{-1} \cdot {}_I\mathbf{r}_{ISO}(G, C, T). \tag{12}$$

Dabei gilt $\gamma = C$ und $\beta = G$.

4. Projektion des Kennfelds in die Ebene des oberen Y-Blockblendenpaares, das auch als *Upper Jaws (UJ)* bezeichnet wird:

$$_C\mathbf{r}_{UJ}(G, C, T) = \begin{pmatrix} {}_C\Delta X_{UJ}(G, C, T) \\ {}_C\Delta Y_{UJ}(G, C, T) \\ {}_C\Delta Z_{UJ}(G, C, T) \end{pmatrix} = k_{UJ}^{-1} \cdot {}_C\mathbf{r}_{ISO}(G, C, T), \tag{13}$$

wobei der Streckfaktor

$$k_{UJ} = \frac{SAD}{SYD} > 1 \tag{14}$$

vom Fokus-Y-Blendenabstand *SYD* abhängt.

5. Projektion des Kennfelds in die Ebene des unteren X-Blockblendenpaares, das auch als *Lower Jaws (LJ)* bezeichnet wird:

$$_C\mathbf{r}_{LJ}(C, C, T) = \begin{pmatrix} {}_C\Delta X_{LJ}(G, C, T) \\ {}_C\Delta Y_{LJ}(G, C, T) \\ {}_C\Delta Z_{LJ}(G, C, T) \end{pmatrix} = k_{LJ}^{-1} \cdot {}_C\mathbf{r}_{ISO}(G, C, T), \tag{15}$$

wobei der Streckfaktor

$$k_{LJ} = \frac{SAD}{SXD} > 1 \tag{16}$$

vom Fokus-X-Blendenabstand *SXD* abhängt.

6. Projektion des Kennfelds in die Ebene des Multi-Leaf-Collimators (MLC):

$$_C\mathbf{r}_{MLC}(G, C, T) = \begin{pmatrix} {}_C\Delta X_{MLC}(G, C, T) \\ {}_C\Delta Y_{MLC}(G, C, T) \\ {}_C\Delta Z_{MLC}(G, C, T) \end{pmatrix} = k_{MLC}^{-1} \cdot {}_C\mathbf{r}_{ISO}(G, C, T), \tag{17}$$

wobei der Streckfaktor

$$k_{MLC} = \frac{SAD}{SMD} > 1 \tag{18}$$

vom Fokus-MLC-Blendenabstand *SMD* abhängt.

7. Bei der Verwendung von Rundcollimatoren entfallen die Schritte 4. bis 6. Die Projektion des globalen Kennfeldes in die Ebene des Rundcollimators oder Circular Cone (CC) wird durch

$$_C\mathbf{r}_{CC}(G, T) = \begin{pmatrix} {}_C\Delta X_{CC}(G, T) \\ {}_C\Delta Y_{CC}(G, T) \\ {}_C\Delta Z_{CC}(G, T) \end{pmatrix} = k_{CC}^{-1} \cdot {}_C\mathbf{r}_{ISO}(G, C = 0°, T) \tag{19}$$

beschrieben, wobei der Streckfaktor

$$k_{CC} = \frac{SAD}{SCD} > 1 \qquad (20)$$

vom Fokus-Cone-Abstand SCD abhängt.

8. Die Positionskorrekturwerte

$$\begin{pmatrix} {}_c\Delta Y_{UJ}(G, C, T) \\ {}_c\Delta X_{LJ}(G, C, T) \\ {}_c\Delta X_{MLC}(G, C, T) \\ {}_c\Delta Y_{MLC}(G, C, T) \end{pmatrix} \in \mathbb{R}^4 \qquad (21)$$

aus den Gleichungen (13), (15) und (17) beziehungsweise

$$\begin{pmatrix} {}_c\Delta X_{CC}(G, T) \\ {}_c\Delta Y_{CC}(G, T) \end{pmatrix} \in \mathbb{R}^2 \qquad (22)$$

aus Gleichung (19) werden auf alle benutzten Collimatorpositionsparameter angewendet. Der vierte Korrekturterm in Gleichung (21) kann jedoch bei keinem der zurzeit im klinischen Einsatz befindlichen Strahlentherapiegeräte mit einem Multi-Leaf-Collimator angewendet werden, da kein Freiheitsgrad dafür vorhanden ist. Dasselbe gilt für die beiden Korrekturterme in Gleichung (22). Geeignete Neukonstruktionen zur Anwendung dieser Korrekturterme wird nachfolgend beschrieben. Schließlich erhält man die korrigierten Positionen der Blockblenden gemäß

$$_cX_{LJ}(G, C, T) = {}_cX_{LJ}|_{RTP} - {}_c\Delta X_{LJ}(G, C, T), \qquad (23)$$

$$_cY_{UJ}(G, C, T) = {}_cY_{UJ}|_{RTP} - {}_c\Delta Y_{UJ}(G, C, T), \qquad (24)$$

des Multi-Leaf-Collimators gemäß

$$_cX_{MLC}(G, C, T) = {}_cX_{MLC}|_{RTP} - {}_c\Delta X_{MLC}(G, C, T), \qquad (25)$$

$$_cY_{MLC}(G, C, T) = {}_cY_{MLC}|_{RTP} - {}_c\Delta Y_{MLC}(G, C, T) \qquad (26)$$

beziehungsweise des verwendeten Rundcollimators gemäß

$$_cX_{CC}(G, T) = {}_cX_{CC}|_{RTP} - {}_c\Delta X_{CC}(G, T), \qquad (27)$$

$$_cY_{CC}(G, T) = {}_cY_{CC}|_{RTP} - {}_c\Delta Y_{CC}(G, T). \qquad (28)$$

In den Gleichungen (23) bis (28) sind die Summanden $(...)|_{RTP}$ die im Bestrahlungsplanungssystem ermittelten Sollpositionen der Blockblenden, der Lamellenpakete beziehungsweise des Rundcollimators eines geometrisch idealen Strahlentherapiegeräts. Die Korrekturen der Lamellenpositionen werden paketweise vorgenommen, das heißt beide Schlitten (Lamellenträger) auf denen die Lamellen und deren Antriebe der *A*- und *B*-Seite (positive und negative *X*-Richtung im Koordinatensystem C) angeordnet sind, führen simultan die Korrekturverschiebungen aus. Bei einem idealen Strahlentherapiegerät mit einem punktförmigen Isozentrum gilt für die Sollpositionen $_cY_{MLC}|_{RTP}$, $_cX_{CC}|_{RTP}$, $_cY_{CC}|_{RTP} \equiv 0$.

9. Für die Ausführung der Korrekturbewegungen durch die Collimatorelemente sind zwei Strategien denkbar:

a. Die winkelabhängigen Korrekturbewegungen in Gleichung (21) beziehungsweise (22) werden offline im Bestrahlungsplanungssystem berechnet, mittels Kontrollpunkten für die tragarmwinkelabhängigen (zeitabhängigen) Blenden- und Lamellenpositionen beziehungsweise Rundcollimatorpositionen definiert und im Onkologie-Informationssystem gespeichert. Die dafür benötigte Rechenzeit ist nebensächlich, da dieser Prozess nicht die Dauer einer Bestrahlungssitzung beeinflusst. Beim Laden der Bestrahlungsparameter aus dem Onkologie-Informationssystem erhält das Strahlentherapiegerät bereits die korrigierten tragarmwinkelabhängigen Blenden- und Lamellenpositionen beziehungsweise Rundcollimatorpositionen.

b. Die winkelabhängigen Korrekturbewegungen werden online vom Strahlentherapiegerät nach dem Laden der Bestrahlungsparameter berechnet. Dazu werden in Echtzeit die notwendigen Verschiebungen aus den Kennfeldern der Gleichungen (13), (15) und (17) beziehungsweise (19) gelesen und auf die momentanen Positionen der Blockblenden und des Multi-Leaf-Collimators beziehungsweise des verwendeten Rundcollimators angewendet. Ein Mikroprozessor mit genügender Rechenleistung ist unabdingbar.

10. Zur Verifikation der jeweiligen Korrektur werden alle drei Isozentroide nochmals gemessen und analysiert.
11. Die Aufnahme des Kennfelds zur Korrektur sowie die Verifikation der Korrektur werden in regelmäßigen Abständen wiederholt, damit die Patientensicherheit trotz sich über der Lebensdauer des Strahlentherapiegeräts ändernder Toleranzbreiten gewährleistet bleibt.

**Durchführung der Positionskorrektur der Strahlungsquelle**

Anstatt der Positionen der Collimatorelemente kann auch die Position der Strahlungsquelle verändert werden, um die räumliche Zentralstrahllage zu korrigieren. Im Photonenbetrieb eines medizinischen Elektronen-Linearbeschleunigers besteht die Strahlungsquelle aus dem Target und dem Ausgleichskörper, die mit ihrem jeweiligen Zentrum in der Zentralstrahlachse liegen (koaxial) und hintereinander angeordnet sind. Photonen können auch ohne Ausgleichskörper auf Krankheitsherde angewendet werden.

Bei dieser Vorgehensweise entfallen bei der im vorherigen Abschnitt beschriebenen Prozedur zur Zentralstrahlkorrektur die Schritte 3. bis 8. und werden durch die folgenden Schritte ersetzt:

3. Ausgehend von Gleichung (11) unter Punkt 2. im vorherigen Abschnitt ergibt sich die Abbildung des globalen Kennfeldes der Zentralstrahlabweichungen ins quellenfeste Koordinatensystem S zu

$$_{S}r_{ISO}(G, C, T) = \mathbf{A}_{\beta}^{-1} \cdot {}_{I}r_{ISO}(G, C, T) \tag{29}$$

mit dem Drehwinkel $\beta = G$.

4. Projektionen des globalen Kennfelds in die Ebenen der Strahlungsquellenkomponenten $S_i$ mit $i \in \{Target, Ausgleichskörper\}$:

$$_{S}r_{S_i}(G, C, T) = \begin{pmatrix} _{S}\Delta X_{S_i}(G, C, T) \\ _{S}\Delta Y_{S_i}(G, C, T) \\ _{S}\Delta Z_{S_i}(G, C, T) \end{pmatrix} = k_{S_i} \cdot {}_{S}r_{ISO}(G, C, T), \tag{30}$$

wobei die Streckfaktoren

$$k_{S_i} = \frac{S_i AD}{S_i AD - S_i MD} \tag{31}$$

Funktionen der Abstände $S_i AD$ und $S_i MD$ der jeweiligen Strahlungsquellenkomponente $S_i$ vom Isozentrum $ISO$ beziehungsweise von der MLC-Ebene sind, falls der Multi-Leaf-Collimator (MLC) zur Strahlformung verwendet wird. Bei der Verwendung von Rundcollimatoren muss in Gleichung (31) der Abstand $S_i MD$ durch den Abstand zur Rundcollimatorebene $S_i CD$ ersetzt werden. Des Weiteren sind bei der Verwendung der X- und Y-Blockblenden die Abstände $S_i XD$ beziehungsweise $S_i YD$ zur jeweiligen Blendenebene einzusetzen. In diesem Fall unterscheiden sich die Streckfaktoren $k_{S_i}$ für die X- und Y-Komponente des Vektors in Gleichung (30).

5. Die Positionskorrekturwerte

$$\begin{pmatrix} _{S}\Delta X_{S_i}(G, C, T) \\ _{S}\Delta Y_{S_i}(G, C, T) \end{pmatrix} \in \mathbb{R}^2 \tag{32}$$

aus Gleichung (30) werden auf die Komponenten der Strahlungsquelle angewendet. Sie besitzen in der Regel einen Freiheitsgrad in der X-Richtung, der eine hohe Positionierungsgenauigkeit besitzt. Der zweite Korrekturterm in Gleichung (32) kann jedoch bei keinem der zurzeit im klinischen Einsatz befindlichen Strahlentherapiegeräte angewendet werden, da kein Freiheitsgrad dafür vorgesehen ist. Eine geeignete Neukonstruktion zur Anwendung dieses Korrekturterms wird weiter unten beschrieben. Schließlich erhält man die korrigierten Positionen der Strahlungsquellenkomponenten zu

$$_sX_{S_i}(G, C, T) = {}_sX_{S_i}|_{RTP} + {}_s\Delta X_{S_i}(G, C, T), \qquad (33)$$

$$_sY_{S_i}(G, C, T) = {}_sY_{S_i}|_{RTP} + {}_s\Delta Y_{S_i}(G, C, T). \qquad (34)$$

Bei einem idealen Strahlentherapiegerät mit einem punktförmigen Isozentrum gilt für die Sollpositionen $_sX_{S_i}|_{RTP}$, $_sY_{S_i}|_{RTP} \equiv 0$.

Die Schritte 1., 2. und 9. bis 11. laufen genauso ab wie es im vorherigen Abschnitt skizziert wurde.

## Durchführung der Patientenpositionskorrektur

Zur Berechnung der Korrekturwerte für die translatorischen Freiheitsgrade des Patientenlagerungstisches benötigt man das Kennfeld in Gleichung (11). Der Korrekturprozess lässt sich in vier Schritte gliedern:

1. Mit der Inversen der Matrix in Gleichung (6) erhält man daraus die Tischkorrekturwerte im tischfesten Koordinatensystem $T$ gemäß

$$_T\mathbf{r}_T(G, C, T + \Delta T) = \begin{pmatrix} _T\Delta X_T(G, C, T + \Delta T) \\ _T\Delta Y_T(G, C, T + \Delta T) \\ _T\Delta Z_T(G, C, T + \Delta T) \end{pmatrix} \in \mathbb{R}^3$$

$$_T\mathbf{r}_T(G, C, T + \Delta T) = \mathbf{A}_\gamma^{-1} \cdot {}_I\mathbf{r}_{ISO}(G, C, T). \qquad (35)$$

Dabei entspricht der Drehwinkel $\gamma = T + \Delta T$ dem momentanen Tischwinkel. Bei der Verwendung eines externen Patientenpositionierungssystems wird ein Korrekturterm $\Delta T \neq 0°$ berücksichtigt, der andernfalls $\Delta T = 0°$ ist. Die korrigierten translatorischen Koordinaten des Patientenlagerungstisches ergeben sich gemäß

$$_TX_T(G, C, T + \Delta T) = {}_TX_T|_{RTP} + {}_T\Delta X_T(G, C, T + \Delta T), \qquad (36)$$

$$_TY_T(G, C, T + \Delta T) = {}_TY_T|_{RTP} + {}_T\Delta Y_T(G, C, T + \Delta T), \qquad (37)$$

$$_TZ_T(G, C, T + \Delta T) = {}_TZ_T|_{RTP} + {}_T\Delta Z_T(G, C, T + \Delta T). \qquad (38)$$

2. Für die Ausführung der Korrekturbewegungen durch den Patientenlagerungstisch kommt nur eine Online-Variante in Betracht, da die Tischkoordinaten über die einzelnen Bestrahlungssitzungen in der Regel nicht konstant sind. Somit können die Korrekturbewegungen meist nicht im Voraus (offline) durch das Bestrahlungsplanungssystem bestimmt werden.

3. Zur Verifikation der jeweiligen Korrektur werden alle drei Isozentroide nochmals gemessen und analysiert.

4. Die Aufnahme des Kennfelds zur Korrektur sowie die Verifikation der Korrektur werden in regelmäßigen Abständen wiederholt, damit die Patientensicherheit trotz sich über der Lebensdauer des Strahlentherapiegeräts ändernder Toleranzbreiten gewährleistet bleibt.

## Konstruktion zur MLC-Positionskorrektur in *Y*-Richtung

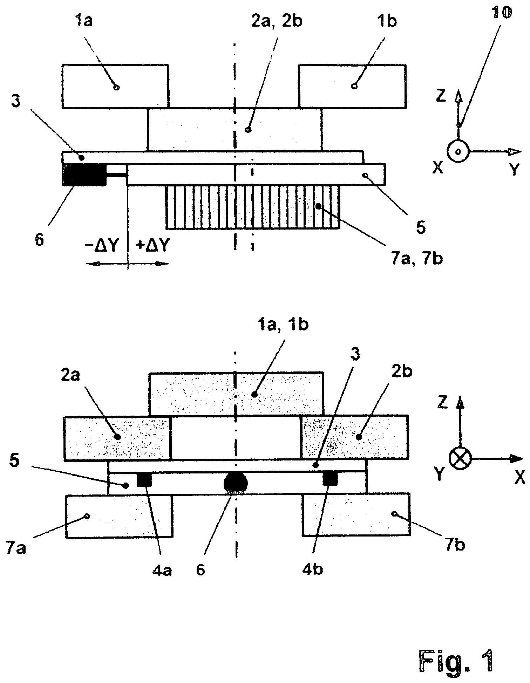

Bei den zurzeit existierenden Strahlentherapiegeräten, die im klinischen Einsatz sind, hat der Multi-Leaf-Collimator (MLC) keinen Freiheitsgrad in der $Y$-Richtung des Collimatorkoordinatensystems C. Eine mögliche Neukonstruktion ist in Figur 1 dargestellt. Zur näheren Erklärung wird in der oberen Ansicht der Figur 1 die Seitenansicht und in der unteren Ansicht die Vorderansicht der Lamellenpakete wiedergegeben.

Zur Erlangung dieses Freiheitsgrades wird die Grundplatte Collimator/MLC, die die mechanische Schnittstelle zwischen dem Zusammenbau der Lower Jaws sowie dem Zusammenbau des MLC darstellt und mit der beide Einheiten fest verschraubt sind, modifiziert. Die neue Grundplatte besteht aus zwei Schichten, die einen Relativfreiheitsgrad in der *Y*-Richtung besitzen. Die fokusseitige Platte 3 wird mit dem Zusammenbau der Lower Jaws 2a und 2b verschraubt; die isozentrumsseitige Platte 5 wird mit dem Zusammenbau des MLC 7a und 7b verschraubt.

Beide Platten sind mithilfe von zwei parallelen Linearführungen 4a und 4b verbunden, die nur ganz kleine Wege zulassen müssen; es reichen wenige Millimeter aus, was die Größenordnung des Korrekturterms $_{C}\Delta Y_{MLC}(G, C, T)$ in Gleichung (21) ist. Als Stellglied für die dynamische beziehungsweise statische Korrekturbewegung könnte beispielsweise ein Piezo-Aktor 6 dienen, der große Kräfte bei kleinen Wegen und großen Geschwindigkeiten erzeugen kann. Er ist fest mit der oberen Grundplatte 3 verbunden und wirkt über eine starre Kupplung auf die untere Grundplatte 5.

Damit die Korrekturwege beziehungsweise -geschwindigkeiten exakt eingestellt und ständig überwacht werden können, erhält dieser Freiheitsgrad zwei voneinander unabhängige Wegmesssysteme. Dafür können beispielsweise lineare Encoder und/oder Potentiometer eingesetzt werden. Ein Linearencoder kann dabei im Piezo-Aktor integriert sein.

**Konstruktion zur Rundcollimatorpositionskorrektur in X- und** *Y*-Richtung

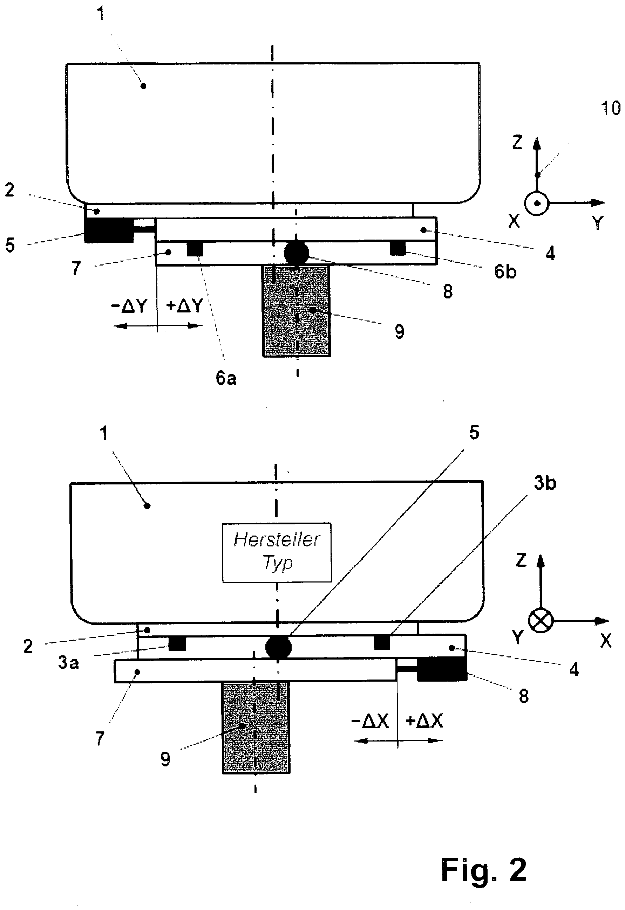

Bei den zurzeit existierenden Strahlentherapiegeräten, die im klinischen Einsatz sind, hat die Halterung der Rundcollimatoren am Strahlerkopf keine Freiheitsgrade in der X- und *Y*-Richtung des Collimatorkoordinatensystems C. Eine mögliche Ausführung der Neukonstruktion beider Freiheitsgrade ist in Figur 2 dargestellt. Die Seitenansicht wird in der oberen Darstellung und die Vorderansicht in der unteren Darstellung der Figur 2 wiedergegeben.

Zur Verwirklichung der Freiheitsgrade wird die Grundplatte des Rundcollimatorhalters dreigeteilt:

- Die Halterung mit der oberen Grundplatte 2 wird nach wie vor fest mit dem Strahlerkopf 1 verschraubt. Mit dem Mittelteil 4 wird sie über das Linearführungspaar 3a und 3b verbunden. Der Piezo-Aktor 5 ist fest mit dem Oberteil 2 verbunden und wirkt über eine starre Kupplung auf den Mittelteil 4.

- Der Mittelteil 4 besitzt nun einen Freiheitsgrad in *Y*-Richtung relativ zum Oberteil 2. Ein weiterer Piezo-Aktor 8 ist wiederum fest mit dem Mittelteil 4 verbunden und wirkt über eine starre Kupplung auf das Unterteil 7, das mithilfe des Linearführungspaars 6a und 6b relativ zum Mittelteil 4 in X-Richtung beweglich ist.

- Das Unterteil 7, das den verwendeten Rundcollimator 9 trägt, besitzt nun den zusätzlichen Freiheitsgrad in X-Richtung.

In Figur 2 befindet sich der *Y*-Freiheitsgrad zur Strahlerkopfseite hin, und der X-Freiheitsgrad zur Rundcollimatorseite hin. Die örtliche Vertauschung beider Freiheitsgrade ist ebenso möglich.

Wie bereits in vorherigen Abschnitt beschrieben wurde, erhält jeder Freiheitsgrad zwei voneinander unabhängige Wegmesssysteme, beispielsweise lineare Encoder und/oder Potentiometer.

**Konstruktion zur Positionskorrektur der Strahlungsquelle in** *Y*-Richtung

Bei den zurzeit existierenden Strahlentherapiegeräten, die im klinischen Einsatz sind, haben die Komponenten der Strahlungsquelle Target und Ausgleichskörper entweder einen Freiheitsgrad in der X- oder einen Freiheitsgrad in der *Y*-Richtung des quellenfesten Koordinatensystems S.

Die Vorgehensweise zur Erlangung des zweiten Freiheitsgrades ist ähnlich wie bei der oben beschriebenen Neukonstruktion des *Y*-Freiheitsgrades des Multi-Leaf-Collimators:

- Modifikation des Schlittens oder Karussells, auf dem die jeweilige Komponente der Strahlungsquelle angeordnet ist. Da das Target und der Ausgleichskörper jeweils einen kreisrunden Querschnitt mit wenigen Zentimetern Durchmesser aufweisen, genügt als Linearführung in der Richtung senkrecht zum bestehenden Freiheitsgrad jeweils ein Langloch mit einer besonders dimensionierten Spielpassung, das möglichst spielfrei die Strahlungsquellenkomponente in der Richtung des bestehenden Freiheitsgrades halten und in der dazu senkrechten Richtung leicht beweglich führen soll. Dazu müssen die vorhandenen Bohrungen durch Fräsen lediglich zu Langlöchern erweitert werden.

- Jeweils ein Piezo-Aktor wird am Schlitten beziehungsweise Karussell jeder Strahlungsquellenkomponente befestigt und wirkt über eine starre Kupplung auf die jeweilige Komponente in der Richtung des neuen Freiheitsgrades.

- Kostengünstiger als die oben beschriebene Spielpassung ist eine Justiereinrichtung für den Piezo-Aktor um die Z-Achse des quellenfesten Koordinatensystems S. Damit kann über die starre Kupplung zwischen dem Piezo-Aktor und der bewegten Komponente die Bewegungsrichtung werkseitig exakt senkrecht und spielfrei zum bereits bestehenden Freiheitsgrad eingestellt werden.

- Damit die Korrekturwege beziehungsweise -geschwindigkeiten exakt eingestellt und ständig überwacht werden können, erhält jeder Freiheitsgrad zwei voneinander unabhängige Wegmesssysteme. Zu diesem Zweck können beispielsweise lineare Encoder und/oder Potentiometer eingesetzt werden.

- Ist eine Komponente $S_i$ der Strahlungsquelle auf einem Karussell angeordnet, sind die anzuwendenden Positionskorrekturwerte, um diejenigen in Gleichung (32) zu erreichen, Funktionen des Drehwinkels ${}_K\gamma_K$ des Karussells um die Z-Achse des karussellfesten Koordinatensystems K, die parallel zu derjenigen des quellenfesten Koordinatensystems S ist, des zusätzlichen radialen Freiheitsgrades ${}_K r_{P_i}$ durch den Piezo-Aktor sowie der Korrekturterme in Gleichung (32). Ist die Karusselldrehachse in negativer $Y$-Richtung vom Zentralstrahl angeordnet, ergibt sich der radiale Korrekturwert für den Piezo-Aktor $P_i$ zu

$$ {}_K\Delta r_{P_i} = \frac{R_K \cdot (1 - \cos\,{}_K\Delta\gamma_K) + {}_S\Delta Y_{S_i}(G,\,C,\,T)}{\cos\,{}_K\Delta\gamma_K} \tag{39} $$

im karussellfesten Koordinatensystem $K$, wobei $R_K$ der Karussellradius ist. Den dazu erforderlichen Drehwinkel des Karussells zur Einstellung der Positionskorrektur in Umfangsrichtung erhält man gemäß

$$ {}_K\Delta\gamma_K = -\arcsin\left[\frac{{}_S\Delta X_{S_i}(G,\,C,\,T)}{R_K + {}_K\Delta r_{P_i}}\right]. \tag{40} $$

Die Gleichungen (39) und (40) bilden ein nichtlineares Gleichungssystem mit den voneinander abhängigen Variablen ${}_K\Delta r_{P_i}$ und ${}_K\Delta\gamma_K$, die Polarkoordinaten darstellen. Durch Einsetzen von Gleichung (39) in (40) erhält man eine einzige nichtlineare Gleichung für die Variable ${}_K\Delta\gamma_K$, die mithilfe des Iterationsverfahrens nach Newton gelöst werden kann. ${}_K\Delta r_{P_i}$ erhält man wiederum durch Einsetzen der Lösung in Gleichung (39). Bei der entgegengesetzten Lage der Karusselldrehachse lauten die Gleichungen zur Bestimmung der Korrekturterme des Piezo-Aktors

$$ {}_K\Delta r_{P_i} = \frac{R_K \cdot (1 - \cos\,{}_K\Delta\gamma_K) - {}_S\Delta Y_{S_i}(G,\,C,\,T)}{\cos\,{}_K\Delta\gamma_K} \tag{41} $$

beziehungsweise

$$ {}_K\Delta\gamma_K = \arcsin\left[\frac{{}_S\Delta X_{S_i}(G,\,C,\,T)}{R_K + {}_K\Delta r_{P_i}}\right]. \tag{42} $$

Hier wurde die Erweiterung um den zusätzlichen Freiheitsgrad in $Y$-Richtung beschrieben. Zur Ergänzung von Freiheitsgraden in $X$-Richtung ist diese Konstruktion ebenfalls geeignet.

**Beschleunigungen bei der Patientenpositionskorrektur**

Die mithilfe der Gleichung (35) ermittelten Korrekturwerte können direkt mittels der Antriebe der translatorischen Tischkoordinaten angewendet werden. Dass dies gefahrlos für den Patienten möglich ist, wird anhand der für einen medizinischen Elektronen-Linearbeschleuniger berechneten Geschwindigkeiten, Beschleunigungen und Rucke (Zeitableitungen der Beschleunigungskomponenten) des Patientenlagerungstisches gezeigt.

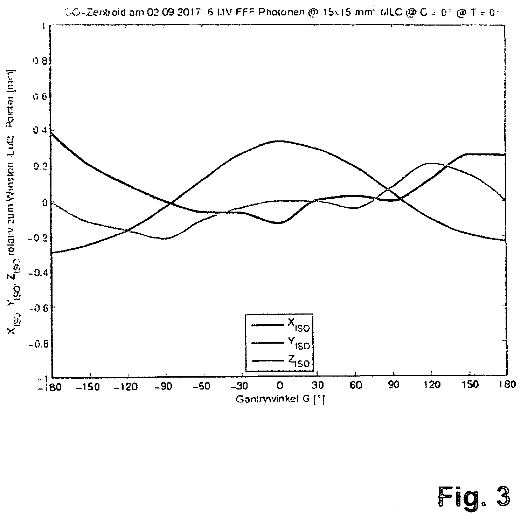

Die Darstellung in Figur 3 zeigt die räumlichen Komponenten der Zentralstrahlabweichung eines medizinischen Elektronen-Linearbeschleunigers bei der Variation des Tragarmwinkels in seinem gesamten Wertebereich. Wiedergegeben sind Abweichungen der räumlichen Zentralstrahlposition vom idealen Isozentrum eines medizinischen Elektronen-Linearbeschleunigers vom Typ Novalis powered by TrueBeamTM STx der Herstellerfirmen BRAINLAB AG (Feldkirchen, Deutschland) und VARIAN Medical Systems, Inc. (Palo Alto, CA, USA) bei der Variation des Tragarmwinkels. Die Berechnung und Grafikerzeugung wurde mittels des Softwarepakets für wissenschaftliches Rechnen MATLAB®, Version R2007a (The MathWorks, Inc., Natick, MA, USA) durchgeführt.

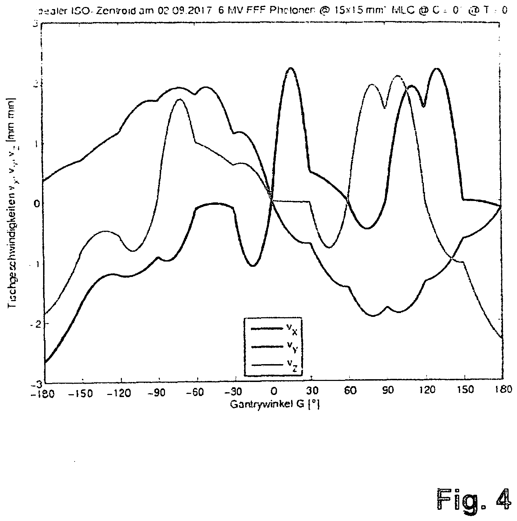

Die Figur 4 zeigt translatorische Geschwindigkeitskomponenten des Patientenlagerungstisches bei der Korrektur der Patientenpositionierung mit den Zentralstrahlabweichungen sowie bei der maximal möglichen Tragarmwinkelgeschwindigkeit von 6°/s.

Die Berechnung und Grafikerzeugung wurde mittels MATLAB®, Version R2007a durchgeführt.

Wie in Figur 4 wiedergegeben, sind bei einer dynamischen Bestrahlungstechnik mit der maximal möglichen Tragarmwinkelgeschwindigkeit *6°/s* alle Beträge der Geschwindigkeitskomponenten < 3 *mm/min*. Im Vergleich dazu werden Patienten bei klinisch üblichen Positionskorrekturbewegungen mit Geschwindigkeiten bis 0.25 m/s = 15 *m/min* bewegt; also mit der 5000-fachen Geschwindigkeit.

Die Komponenten der Tischbeschleunigungen haben Beträge < 5 $\mu m/s^2$. Auf einen Patienten mit einer durchschnittlichen Körpermasse von *m* = 75 kg würde demnach die maximale Trägheitskraft

$$F_{max} = -m \cdot a_{max} = -75 \ kg \cdot 5 \ \mu m/s^2 = -0.375 \ mN \tag{43}$$

betragen. Ein Körper mit der Masse 38 mg würde eine Gewichtskraft dieses Betrags erfahren. Bei nochmaliger Ableitung nach der Zeit erhält man die Komponenten der Tischrucke, deren Beträge allesamt < 2.5 $\mu m/s^3$ sind.

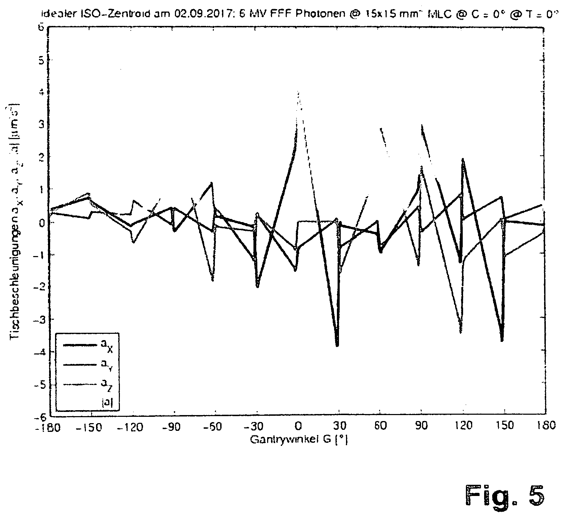

Die grafische Darstellung der Figur 5 zeigt die translatorischen Beschleunigungskomponenten und die resultierende Beschleunigung des Patientenlagerungstisches bei der Korrektur der Patientenposition mit den Zentralstrahlabweichungen nach Figur 3 sowie bei der maximal möglichen Tragarmwinkelgeschwindigkeit von 6°/s. Die Berechnung und Grafikerzeugung wurde mittels MATLAB®, Version R2007a durchgeführt.

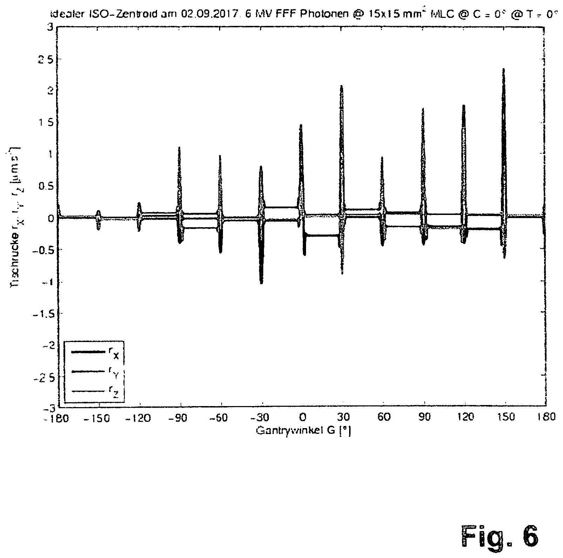

Mit der Darstellung der Figur 6 werden die translatorischen Ruckkomponenten des Patientenlagerungstisches bei der Korrektur der Patientenposition mit den Zentralstrahlabweichungen nach Figur 3 wiedergegeben. Auch hierbei beträgt die Tragarmwinkelgeschwindigkeit 6°/s (Worst Case).

Ein Patient auf dem Patientenlagerungstisch würde die zur Positionskorrektur benötigten geringen Beschleunigungen kaum spüren. Bei einer guten, reproduzierbaren Lagerung mittels geeigneter Lagerungshilfen besteht zudem keine Gefahr einer Relativbewegung zwischen dem Patienten und der Lagerungstischplatte.

## Anwendung der Korrekturbewegungen im Strahlentherapiegerät

Die Anwendung der Korrekturbewegungen zur geometrischen Zentralstrahlkorrektur mittels der Collimatorelemente Blockblenden, Lamellenpakete und Rundcollimatoren ist sowohl statisch als auch dynamisch möglich. Ihre Antriebe sind dynamikfähig, das heißt sie können definierte zeitabhängige Bewegungen in Rahmen ihrer Positioniergenauigkeit ausführen. Sie besitzen jeweils ein redundantes Wegmesssystem. Die jeweiligen Primär- und Sekundärpositionen werden ständig überwacht. Es müssen lediglich die winkel- beziehungsweise zeitabhängigen Korrekturbewegungen an die Antriebsregelungen der Collimatorelemente gegebenen werden.

Bei der Verwendung der Patientenpositionskorrektur erhalten die translatorischen Tischkoordinatenantriebe die Bewegungsbefehle. Sie besitzen ebenfalls redundante Wegmesssysteme, wobei das oben Gesagte ebenfalls gilt. Bei den nicht zur stereotaktischen Radiotherapie und Radiochirurgie geeigneten Strahlentherapiegeräten müsste lediglich die Positioniergenauigkeit der Tischkoordinaten um den Faktor 10 gesteigert werden, damit Wegdifferenzen von ≤ 0.1 mm angewendet werden können.

Zum besseren Verständnis über die Umsetzung der Korrekturbewegungen werden die Regelkreise der relevanten Antriebe nachfolgend näher beschrieben. Dort wird auch diskutiert, welche regelungstechnischen Erweiterungen dafür notwendig sind.

## Regelung der Antriebe

Moderne Strahlentherapiegeräte besitzen geregelte Antriebe, die sowohl statisch als auch dynamisch vorgegebene Positionen von translatorischen und rotatorischen Freiheitsgraden anfahren können. Hier werden nun die für die geometrische Zentralstrahlkorrektur sowie für die Patientenpositionskorrektur benötigten Regelungsmechanismen beschrieben.

## 1. Positionierungsgenauigkeiten

Der für radiochirurgische Anwendungen konzipierte medizinische Linearbeschleuniger TrueBeam™ STx (Varian Medical Systems, Inc., Palo Alto, CA, USA) weist beispielsweise Antriebe mit den in Tabelle 4 zusammengefassten Positionierungsgenauigkeiten und Geschwindigkeitsbereichen auf. Ihre Leistungsfähigkeit bezüglich der benötigten Korrekturgeschwindigkeiten in der Größenordnung von 3 *mm/min* = 5·10⁻³ *cm/s* ist um ein Vielfaches größer und damit völlig ausreichend. Ihre jeweilige Positioniergenauigkeit müsste jedoch für die Umsetzung der Erfindung um einen Faktor 5 ... 10 verbessert werden.

**Tabelle 4:** Positionierungsgenauigkeiten, -reproduzierbarkeiten und Geschwindigkeitsbereiche der Collimator- und Tischantriebe eines Elektronen-Linearbeschleunigers vom Typ TrueBeam™ STx.

| Komponente | Positionierungsgenauigkeit [mm] | Positionierungsreproduzierbarkeit [mm] | Geschwindigkeitsbereich [cm/s] |
|---|---|---|---|
| X-Blockblenden | ±1 | ±0.5 | 0...2.5 |
| Y-Blockblenden | ±1 | ±0.5 | 0...2.5 |
| MLC-Schlitten | ±1 | ±0.5 | 0...1.2 |
| MLC-Lamellen | ±1 | ±0.5 | 0...2.5 |
| X-Tischachse | ±0.5 | ±0.5 | 0...3.5 |
| Y-Tischachse | ±0.5 | ±0.5 | 0...7 |
| Z-Tischachse | ±0.5 | ±0.5 | 0...2 |

## 2. Messsysteme und Feedback der Regelkreise

Alle Freiheitsgrade, die zur Korrektur der Zentralstrahlabweichungen benötigt werden, besitzen serienmäßig redundante Messsysteme - jeweils ein primäres und ein sekundäres - sowie einen eigenen geschlossenen Regelkreis, wobei die Positions- beziehungsweise Geschwindigkeitsmesswerte an den Regler rückgeführt werden. Dieses Feedback schließt den Regelkreis.

Die im Elektronen-Linearbeschleuniger TrueBeam™ STx verwendeten Messsysteme sind Resolver, Encoder und Potentiometer. Resolver sind elektrische Umformer, die Drehwinkel relativ zu einer bekannten physikalischen Position eines Freiheitsgrades messen. Diese Referenzposition ist hier ein mechanischer Anschlag der entsprechenden Bewegungsachse. Encoder konvertieren rotatorische beziehungsweise translatorische Positionen in digitale Signale; sie messen absolute Positionen. Potentiometer sind elektrische Widerstände, deren Werte in linearer Weise winkel- beziehungsweise wegabhängig sind. Die hier eingesetzten Messsysteme werden in Tabelle 5 zusammengestellt.

| Komponente | primäres System | sekundäres System | tertiäres System |
|---|---|---|---|
| X-Blockblenden | Resolver | Resolver | - |
| Y-Blockblenden | Resolver | Resolver | - |
| MLC-Schlitten | magnetischer Encoder | optischer Encoder | - |
| MLC-Lamellen | magnetischer Encoder | lineares Encoder | - |
| X-Tischachse | Encoder | Encoder | Resolver |
| Y-Tischachse | Encoder | Encoder | Resolver |
| Z-Tischachse | absolutes Potentiometer | absolutes Potentiometer | Resolver |

Tabelle 5: Redundante Messsysteme der Collimator- und Tischantriebe eines Elektronen-Linearbeschleunigers vom Typ TrueBeam™ STx.

## 3. Überwachung der Antriebe

Die primären Sensoren überwachen die geregelten Antriebe. Die Rückführung der Messsignale im jeweiligen Regelkreis dient der Initialisierung und Überwachung der entsprechenden Positionierung und Bewegung. Bei einer zielgerichteten Bewegung während eines dynamischen Bestrahlungsablaufs startet das Hauptsteuerprogramm, das kurz Supervisor genannt wird, die dazugehörige Bewegung automatisch. Der Supervisor synchronisiert dabei die Abgabe der Energiedosis mit der Bewegung. Die Zykluszeit der Regelung und Synchronisierung beträgt hier 10 *ms.*

Vergleicht man diese Zeitdauer mit der Periodendauer der zeitlichen Isozentrumsabweichungen, die bei einer maximaler Tragarmwinkelgeschwindigkeit ungefähr 30 *s* beträgt (vergleiche Figur 3), wird deutlich, dass eine Korrektur der Isozentrumsabweichungen in Echtzeit möglich ist.

### 4. Ablauf der Regelvorgänge

Nachdem der Bestrahlungsplan mit allen Parametern vom Strahlentherapiegerät geladen wurde, berechnet der Supervisor die Trajektorien aller bewegten Achsen bezüglich Abfolge, Geschwindigkeit und Dauer. Der Supervisor generiert die Befehle für die Datenknoten der Antriebe, die als Nodes bezeichnet werden. Die Befehle werden in Zeitabständen von 10 *ms* aktualisiert und beinhalten zwei zukünftige Positionswerte, die in weiteren 10 *ms* beziehungsweise 20 *ms* erreicht werden sollen. Die Nodes wiederum generieren die Bewegungsvorgaben für die ihnen untergeordneten Antriebe und benötigen dazu weniger als eine Zeitdauer von 10 *ms.* Schließlich können damit die Antriebe die geforderten Bewegungen ausführen.

### 5. Kommunikation zwischen Supervisor und Nodes

Der Datenaustausch erfolgt bei jedem einzelnen Synchronisationspuls. Alle 10 *ms* werden von jedem Node die von den Sensoren erhaltenen Positionswerte mit dem jeweiligen Sollwert verglichen. Der Supervisor überprüft die Positionswerte der Nodes und berechnet jeweils zwei zukünftige Positionswerte. Für die Umsetzung der Erfindung sind drei Nodes wichtig, die beim TrueBeam™ STx *COL, CCHL* und *CCHU* heißen. Sie sind für die Bewegungen aller Collimatorelemente, der vertikalen Tischkoordinate ($Z$) beziehungsweise der lateralen ($X$) und longitudinalen Tischkoordinate ($Y$) zuständig.

### 6. Regelung durch sich zusätzlich verstärkende Mechanismen

Hierbei wird das Fehlererkennungsvermögen der redundanten Messsysteme benutzt. Bei Bedarf werden Justierungen vorgenommen, um Positionen, Geschwindigkeiten und Trajektorien zu korrigieren. Das passiert im jeweiligen Node, indem der Befehl des Supervisors mit der aktuellen Position des primären Sensors des betroffenen Antriebs verglichen wird.

### 7. Notwendige Erweiterungen der Regelkreise

Die Regelungen der Antriebe zur Bewegung der Blockblenden, der MLC-Schlitten sowie der Lamellen sind bereits dynamikfähig; sie können außer Positionen auch Geschwindigkeiten regeln. Möchte man die vorgeschlagene Patientenpositionskorrektur anwenden, müssen die Regelkreise aller drei Tischkoordinaten um jeweils eine Geschwindigkeitsregelung erweitert werden. Dasselbe gilt auch für die vorhandenen Antriebe der Komponenten der Strahlungsquelle; ihre Ansteuerung muss um jeweils eine Geschwindigkeitsregelung erweitert werden.

Zur Erhöhung der Positioniergenauigkeit aller Antriebe sollte das jeweilige Spiel durch modifizierte Maschinenelemente verringert werden. Dies sind vorgespannte Wälzlagerungen, Kugelumlaufspindeln, geringere Toleranzbreiten bei Passungen sowie zweigeteilte Ritzel mit Federvorspannung. Das Wegauflösungsvermögen der Sensoren ist ausreichend; es beträgt 0.1 *mm* bezogen auf die Isozentrumsebene.

### Das Neue der Erfindung

Durch die Verwendung aller geometrischen Informationen über die exakt vermessenen Isozentroide eines Strahlentherapiegeräts ist man in der Lage, die Korrekturterme für die strahlformenden Collimatorelemente und/oder für die translatorischen Freiheitsgrade des Patientenlagerungstisches zu berechnen; dies geschieht offline durch das Bestrahlungsplanungssystem beziehungsweise online durch das Strahlentherapiegerät. Diese Korrekturterme können bei jeder Bestrahlungssitzung in Echtzeit angewendet werden. Man hat keinerlei Beschränkungen bei der Wahl optimaler Bestrahlungsparameter, wovon eine gravierende in [2] beschrieben wird; der Collimatorwinkel C = 90° = const. ist nicht immer ein optimaler Bestrahlungsparameter.

### Vorteile gegenüber dem Stand der Technik

Bei der vorliegenden Erfindung wird keine teure Hardware-Lösung angewendet, um die Tragarmsteifigkeit weiter zu erhöhen, damit die Zentralstrahlpositionsabweichungen merklich kleiner werden. Der Tragarm eines Elektronen-Linearbeschleunigers ist ein sehr komplexes Bauteil, das viele Komponenten der Teilchenbeschleunigung, Strahlerzeugung, Energieversorgung und Regelungstechnik aufnehmen muss. Mit einer typischen Bauteilgesamtmasse von 7.2 t hat man bereits ein Optimum zwischen der Bauteilsteifigkeit und dem Eigengewicht erreicht.

Die vorgeschlagene Lösung macht es möglich, die Positionsabweichungen zwischen dem realen Zentralstrahl und dem Tumorzentrum während der Patientenbestrahlung in Abhängigkeit der Bestrahlungsparameter Tragarm-, Collimator- und Patientenlagerungstischwinkel in Echtzeit zu korrigieren. Man kann mit aus radioonkologischer Sicht

# EP 3 877 049 B1

optimalen Bestrahlungsparametern die Energiedosis mit der höchstmöglichen geometrischen Präzision im Krankheitsherd deponieren.

Gebrauchte Strahlentherapiegeräte ohne präzises Isozentrum können mithilfe der Erfindung Stereotaxiefähigkeit erlangen. Zukünftig können Strahlentherapiegeräte mit einem Tragarm wieder leichter gebaut werden.

## Literatur

[1] Dubbel - Taschenbuch für den Maschinenbau. 15. korrigierte und ergänzte Auflage, herausgegeben von W. Beitz und K.-H. Küttner, Springer-Verlag, Berlin, Heidelberg, New York, Tokyo, 1986.

[2] Du, W., Gao, S., Wang, X., Kudchadker, R.J.: Quantifying the gantry sag on linear accelerators and introducing an MLC-based compensation strategy. Medical Physics, 39(4), 2156-2162, 2012.

[3] Pfeiffer, F., Glocker, Ch.: Multibody Dynamics with Unilateral Contacts. Series Editors: Ali H. Nayfeh and Arun V. Holden. John Wiley & Sons, Inc., New York, 1996.

Bezugszeichen

| | |
|---|---|
| 1 | Strahlerkopf |
| 1a | Y1-Blockblende |
| 1b | Y2-Blockblende |
| 2 | Oberteil der Rundcollimatorhalterung |
| 2a | X1-Blockblende |
| 2b | X2-Blockblende |
| 3 | Oberteil der Grundplatte Collimator/MLC |
| 3a | linksseitige Linearführung für den Freiheitsgrad in Y-Richtung |
| 3b | rechtsseitige Linearführung für den Freiheitsgrad in Y-Richtung |
| 4 | Mittelteil der Rundcollimatorhalterung |
| 4a | linksseitige Linearführung |
| 4b | rechtsseitige Linearführung |
| 5 | Piezo-Aktor für die Bewegungen $\pm\Delta Y$ |
| 6 | Piezo-Aktor für die Bewegungen $\pm\Delta Y$ |
| 6a | vorderseitige Linearführung für den Freiheitsgrad in X-Richtung |
| 6b | rückseitige Linearführung für den Freiheitsgrad in X-Richtung |
| 7 | Unterteil der Rundcollimatorhalterung |
| 7a | Lamellenpaket der B-Seite beziehungsweise X1-Seite |
| 7b | Lamellenpaket der A-Seite beziehungsweise X2-Seite |
| 8 | Piezo-Aktor für die Bewegungen $\pm\Delta X$ |
| 9 | Rundcollimator |
| 10 | collimatorfestes Koordinatensystem C mit den Achsen X, Y und Z |
| 11 | Unterteil der Grundplatte Collimator/MLC mit dem neuen Freiheitsgrad in Y-Richtung |

## Patentansprüche

**1.** Verfahren zur echtzeitbezogenen Korrektur der räumlichen Lage des Zentralstrahles von Strahlentherapiegeräten durch Änderung der Positionen von Collimatorelementen und/oder der räumlichen Lage des Krankheitsherdzentrums durch Änderung der Position des Patientenlagerungstisches, **dadurch gekennzeichnet, dass**

a. während des Therapieverlaufes auftretende räumliche Positionsänderungen der Collimatorelemente und/oder des Patientenlagerungstisches messtechnisch erfasst werden,

b. die echtzeitbezogenen Berechnungen der dafür erforderlichen Korrekturbewegungen außerhalb des Strahlentherapiegeräts im Bestrahlungsplanungssystem und/oder in mindestens einem Mikrocontroller der Steuerung des Strahlentherapiegeräts durchgeführt werden und

c. die Änderungen derer erfassten räumlichen Positionen in ihre vorgegebene Sollposition jeweils über mit dem Collimator und/oder dem Patientenlagerungstisch verbundene separate Verstelleinrichtungen in Echtzeit vorgenommen werden.

2. Verfahren nach Patentanspruch 1, **dadurch gekennzeichnet, dass** die Steuerung der Positionsänderungen von jeweils einer Steuerbaugruppe ausgeführt wird, die als ein Microcontroller ausgebildet ist, der ein die Verstelleinrichtungen steuerndes Computerprogramm enthält.

3. Verfahren nach Patentanspruch 1, **dadurch gekennzeichnet, dass** mittels der zuschaltbaren Steuerbaugruppen die Steuerung des Collimators innerhalb der Freiheitsgrade in X- und Y-Richtung des collimatorfesten Koordinatensystems und/oder des Patientenlagerungstisches innerhalb der Freiheitsgrades in X-, Y- und Z-Richtung des tischfesten Koordinatensystems erfolgt.

4. Verfahren nach den Patentansprüchen 1 und 3, **dadurch gekennzeichnet, dass** die Steuerung innerhalb der Freiheitsgrade in X- und Y-Richtung auf die echtzeitbezogene Positionsänderung der Collimatorelemente beschränkt ist.

5. Verfahren nach den Patentansprüchen 1 und 3, **dadurch gekennzeichnet, dass** die Steuerung innerhalb der Freiheitsgrade in X-, Y- und Z-Richtung auf die echtzeitbezogene Positionsänderung des Patientenlagerungstisches beschränkt ist.

6. Verfahren nach den Patentansprüchen 1 und 3, **dadurch gekennzeichnet, dass** die Steuerung innerhalb der Freiheitsgrade durch kombinierte echtzeitbezogene Positionsänderungen der Collimatorelemente und des Patientenlagerungstisches erfolgt.

7. Verfahren nach Patentanspruch 1, **dadurch gekennzeichnet, dass** die Steuerbaugruppe optional nach Genauigkeit der Positionsänderung, Art des Freiheitsgrades und/oder der Auswahl oder Kombination der zu steuernden Collimatorelemente und des Patientenlagerungstisches voreinstellbar ausgebildet ist.

8. Verfahren nach Patentanspruch 1, **dadurch gekennzeichnet, dass** bei der Steuerung und den Positionsänderungen der Collimatorelemente und/oder des Patientenlagerungstisches translatorische und/oder rotatorische Freiheitsgrade der Bewegungen einbezogen werden.

9. Verfahren nach den Patentansprüchen 1 und 4, **dadurch gekennzeichnet, dass** die in Y-Richtung gesteuerten Positionsänderungen des Multi-Leaf-Collimators sowie die in X- und Y-Richtung gesteuerten Positionsänderungen des Rundcollimators mittels Piezo-Aktoren ausgefübrt werden.

10. Verfahren nach den Patentansprüchen 1 und 2, **dadurch gekennzeichnet, dass** der programmtechnische Ablauf und die Schnittstelle des im Microcontroller implementierten Computerprogrammes mit denen des Hauptprogramms des Strahlentherapiegeräts kompatibel gestaltet sind.

11. Verfahren nach den Patentansprüchen 1, 2, 3, 4, 5, 6, 7, 8 und 10, **dadurch gekennzeichnet, dass** anstatt der Änderung der Positionen von Collimatorelementen die Position der Strahlungsquelle innerhalb der Freiheitsgrade in X- und Y-Richtung des quellenfesten Koordinatensystems verändert wird.

12. Verfahren nach Patentanspruch 11, **dadurch gekennzeichnet, dass** die Position einer aus einem Target und einem Ausgleichskörper bestehenden Strahlungsquelle eines im Photonenbetrieb befindlichen Strahlentherapiegeräts verändert wird.

13. Verfahren nach den Patentansprüchen 1, 4, 9 und 11, **dadurch gekennzeichnet, dass** die in X-und Y-Richtung gesteuerten Positionsänderungen der Strahlungsquelle mittels Piezo-Aktoren ausgeführt werden.

14. Verfahren nach Patentanspruch 13, **dadurch gekennzeichnet, dass** die Positionsänderungen einer auf einem Karussell angeordneten Strahlenquelle durch kombinierte Bewegungen des Karussells und des Piezo-Aktors durchgeführt werden.

15. Verfahren nach den Patentansprüchen 1 und 11, **dadurch gekennzeichnet, dass** der Elektronenstrahl vor dem Auftreffen auf das Target durch Umlenkmagnete und Steuerspulen synchron mit den Positionsänderungen des Targets gesteuert wird, sodass der Elektronenstrahl stets auf das Targetzentrum trifft.

## Claims

1. Method for the real-time-related correction of the spatial position of the central beam of radiation therapy devices by changing the positions of collimator elements and/or of the spatial position of the center of disease foci by changing the position of the patient support table, **characterized in that**

   a. spatial changes in the position of the collimator elements and/or and/or the patient support table occurring during the course of therapy are measured,

   b. the real-time-related calculations of the corrective movements required for this are carried out outside the radiation device in the irradiation planning system and/or in at least one microcontroller of the control system of the radiation therapy device, and

   c. the changes of those recorded spatial positions regarding their predetermined target position each is carried out via separate adjustment devices connected to the collimator and/or the patient support table in real time.

2. Method according to claim 1, **characterized in that** the control of the changes in the position is carried out by a respective control module, which is designed as a microcontroller, which contains a computer program controlling the adjustment devices.

3. Method according to claim 1, **characterized in that,** by means of the connectable control modules, the control of the collimator is effected within the degrees of freedom in the X and Y directions of the collimator-fixed coordinate system and/or of the patient support table is effected within the degrees of freedom in the X, Y and Z directions of the table-fixed coordinate system.

4. Method according to claims 1 and 3, **characterized in that** the control within the degrees of freedom in the X and Y directions is limited to the real-time related position change of the collimator elements.

5. Method according to claims 1 and 3, **characterized in that** the control within the degrees of freedom in the X, Y and Z directions is limited to the real-time related position change of the patient support table.

6. Method according to claims 1 and 3, **characterized in that** the control within the degrees of freedom is effected by combined real-time related position changes of the collimator elements and the patient support table.

7. Method according to claim 1, **characterized in that** the control module is optionally designed to be pre-adjustable according to the accuracy of the position change, the type of degree of freedom and/or the selection or combination of the collimator elements to be controlled and the patient support table.

8. Method according to claim 1, **characterized in that** translational and/or rotatory degrees of freedom of the movements are included in the control and the position changes of the collimator elements and/or the patient support table.

9. Method according to claims 1 and 4, **characterized in that** the position changes of the multi-leaf collimator controlled in the Y direction and the position changes of the round collimator controlled in the X and Y directions are carried out by means of piezo actuators.

10. Method according to claims 1 and 2, **characterized in that** the programming sequence and the interface of the computer program implemented in the microcontroller are designed to be compatible with those of the main program of the radiation therapy device.

11. Method according to claims 1, 2, 3, 4, 5, 6, 7, 8 and 10, **characterized in that,** instead of changing the positions of collimator elements, the position of the radiation source is changed within the degrees of freedom in the X and Y directions of the source-fixed coordinate system.

12. Method according to claim 11, **characterized in that** the position of a radiation source consisting of a target and a compensating element of a radiation therapy device in photon mode is changed.

13. Method according to claims 1, 4, 9 and 11, **characterized in that** the position changes of the radiation source controlled in the X and Y directions are carried out by means of piezo actuators.

14. Method according to claim 13, **characterized in that** the position changes of a radiation source arranged on a carousel are carried out by combined movements of the carousel and the piezo actuator.

15. Method according to claims 1 and 11, **characterized in that** before striking the target the electron beam is controlled by deflecting magnets and control coils synchronously with the position changes of the target, such that the electron beam always strikes the target center.

**Revendications**

1. Procédé permettant la correction en temps réel de la position spatiale du rayon central d'appareils de radiothérapie en modifiant des positions d'éléments collimateurs et/ou de la position spatiale du centre de foyer d'infection en modifiant la position de la table de support pour patient, **caractérisé en ce que**

   a. les modifications de position spatiale des éléments collimateurs et/ou de la table de support pour patient survenant pendant le processus de thérapie sont détectés en termes de la technique de mesure ;
   b. les calculs en temps réel des déplacements de correction nécessaires à cet effet sont exécutés à l'extérieur de l'appareil de radiothérapie dans le système de planification d'irradiation et/ou dans au moins un microdispositif de commande de la commande de l'appareil de radiothérapie ; et
   c. les modifications de leurs positions spatiales détectées vers leur position de consigne prédéfinie sont respectivement effectuées en temps réel par l'intermédiaire de dispositifs de réglage séparés connectés au collimateur et/ou à la table de support pour patient.

2. Procédé selon la revendication 1, **caractérisé en ce que** la commande des modifications de position est exécutée par un module de commande respectif réalisé comme un microdispositif de commande qui contient un programme informatique commandant les dispositifs de réglage.

3. Procédé selon la revendication 1, **caractérisé en ce que** la commande du collimateur est exécutée à l'intérieur des degrés de liberté dans les directions X et Y du système de coordonnées fixe du collimateur et/ou de la table de support pour patient à l'intérieur des degrés de liberté dans les directions X, Y et Z du système de coordonnées fixe de la table au moyen des modules de commande pouvant être mis en circuit.

4. Procédé selon les revendications 1 et 3, **caractérisé en ce que** la commande à l'intérieur des degrés de liberté dans les directions X et Y est limitée à la modification de position en temps réel des éléments collimateurs.

5. Procédé selon les revendications 1 et 3, **caractérisé en ce que** la commande à l'intérieur des degrés de liberté dans les directions X, Y et Z est limitée à la modification de position en temps réel de la table de support pour patient.

6. Procédé selon les revendications 1 et 3, **caractérisé en ce que** la commande est exécutée à l'intérieur des degrés de liberté par des modifications de position combinées en temps réel des éléments collimateur et de la table de support pour patient.

7. Procédé selon la revendication 1, **caractérisé en ce que** le module de commande est configuré de manière à pouvoir être éventuellement préréglé en fonction d'une précision de la modification de position, d'un type de degré de liberté et/ou d'une sélection ou d'une combinaison des éléments collimateurs et de la table de support pour patient à commander.

8. Procédé selon la revendication 1, **caractérisé en ce que,** lors de la commande et des modifications de position des éléments collimateurs et/ou de la table de support pour patient, des degrés de liberté en translation et/ou en rotation des déplacements sont pris en compte.

9. Procédé selon les revendications 1 et 4, **caractérisé en ce que** les modifications de position du collimateur multilame commandées dans la direction Y ainsi que les modifications de position du collimateur circulaire commandées dans les directions X et Y sont effectuées au moyen d'actionneurs piézoélectriques.

10. Procédé selon les revendications 1 et 2, **caractérisé en ce que** le déroulement technique de programme et l'interface du programme informatique mis en œuvre dans le microdispositif de commande sont configurés de manière à être compatibles avec ceux du programme principal de l'appareil de radiothérapie.

**11.** Procédé selon les revendications 1, 2, 3, 4, 5, 6, 7, 8 et 10,
**caractérisé en ce que,** au lieu de modifier les positions d'éléments collimateurs, la position de la source de rayonnement à l'intérieur des degrés de liberté dans les directions X et Y du système de coordonnées fixe de la source est modifiée.

**12.** Procédé selon la revendication 11, **caractérisé en ce que** la position d'une source de rayonnement constituée d'une cible et d'un corps de compensation d'un appareil de radiothérapie à fonctionnement photonique présent est modifiée.

**13.** Procédé selon les revendications 1, 4, 9 et 11, **caractérisé en ce que** les modifications de position de la source de rayonnement commandées dans les directions X et Y sont effectuées au moyen d'actionneurs piézoélectriques.

**14.** Procédé selon la revendication 13, **caractérisé en ce que** les modifications de position d'une source de rayonnement disposée sur un carrousel sont effectuées par des déplacements combinés du carrousel et de l'actionneur piézoélectrique.

**15.** Procédé selon les revendications 1 et 11, **caractérisé en ce que** le rayon d'électrons, avant d'atteindre la cible, est commandé par des aimants de déviation et des bobines de commande de manière synchrone avec les modifications de position de la cible, de sorte que le rayon d'électrons atteint toujours le centre de la cible.

Fig. 1

Fig. 2

Fig. 3

Fig. 4

26

idealer ISO–Zentroid am 02.09.2017: 6 MV FFF Photonen @ 15x15 mm² MLC @ C = 0° @ T = 0°

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2883568 A1
- DE 10147633 A1
- DE 102010041752 B4
- WO 2013041720 A1
- EP 1740098 B1
- DE 202012013430 U1
- DE 102011082257 B4

**In der Beschreibung aufgeführte Nicht-Patentliteratur**

- **VON W. BEITZ** ; **K.-H. KÜTTNER**. Dubbel - Taschenbuch für den Maschinenbau. Springer-Verlag, 1986, vol. 15
- **DU, W.** ; **GAO, S.** ; **WANG, X.** ; **KUDCHADKER, R.J.** Quantifying the gantry sag on linear accelerators and introducing an MLC-based compensation strategy. *Medical Physics*, 2012, vol. 39 (4), 2156-2162
- **PFEIFFER, F.** ; **GLOCKER, CH.** Multibody Dynamics with Unilateral Contacts. Series. John Wiley & Sons, Inc., 1996